# EUROPÄISCHE PATENTANMELDUNG

(11) **EP 1 190 667 A2**
(43) Veröffentlichungstag der Anmeldung: **27.03.2002**
(21) Anmeldenummer: 01122781.6
(22) Anmeldetag: 21.09.2001
(51) Int. Cl.: A61B 3/12

(54) **System zur Aufnahme des Netzhautreflexbildes**

(30) Priorität: 23.09.2000 DE 10047237
(71) Anmelder: Physoptics Opto-Electronic GmbH, 82319 Starnberg (DE)
(72) Erfinder: Eberl, Heinrich A., 87463 Probstried (DE); Eberl, Roland H. C., 80687 München (DE); Brandl, Hans Dr. Med., 83093 Bad Endorf (DE)
(74) Vertreter: Winter, Brandl, Fürniss, Hübner, Röss, Kaiser, Polte Partnerschaft

(57) **Zusammenfassung**

Beschrieben wird ein System zur Aufnahme des Netzhautreflexbildes mittels eines Scansystems zum Abtasten eines Bildes auf der Netzhaut und zur Eispeisung von optischen Zusatzsignalen in das Auge, bei dem ein Strahlenteiler zur Transmission von Strahlen der Außenwelt in das Auge und zur Reflexion der von der Netzhaut des Auges zurückgestreuten Strahlen, eine Empfangseinheit zur Aufnahme der zurückgestreuten Strahlen und eine Projektionseinheit zur Projektion von Lichtstrahlen in das Auge verwendet werden, um auf der Netzhaut eine Abbildung zu erzeugen, die sich dem ursprünglich auf der Netzhaut abgebildeten Bild überlägert. Das Scansystem lenkt beim Abtasten die von der Netzhaut kommenden Strahlen um und weiter zu einem opto-elektronischen Detektor zur seriellen Aufnahme des Netzhautreflexes. Das System zeichnet sich dadurch aus, dass es derart ausgestaltet ist, dass es auf dem Gebiet der Medizintechnik, der Ophthalmolgie, der Strabologie und der Neuroophthalmolgie die Funktion von Spezialgeräten übernehmen kann.

## Beschreibung

Die Erfindung betrifft ein System auf der Basis einer Vorrichtung zur Erfassung des auf ein Auge fallenden Lichtbildes.

Aus den deutschen Offenlegungsschriften DE 196 31 414 A1 und DE 197 28 890 sind optische Vorrichtungen bekannt, die eine Aufnahme des Netzhautreflexbildes und eine Überlagerung von Zusatzbildern im Auge ermöglichent.

Da die in den obengenannten Anmeldungen beschriebenen Vorrichtungen und Systeme bevorzugt in Form einer Brille ausgestaltet sind, werden sie nachfolgend der Einfachheit halber auch als Brillensystem bezeichnet. Diese Bezeichnung impliziert keine Einschränkung. Selbstverständlich sind auch andere Ausführungsformen solcher Vorrichtungen und Systeme in den unten beschriebenen Zusammenhängen anstelle des "Brillensystems" gleichfalls anwendbar.

In der DE 196 31 414 A1 werden zahlreiche Anwendungsmöglichkeiten des darin offenbarten Brillensystems angesprochen, ohne daß diese genauer beschrieben werden konnten.

Die Aufgabe der Erfindung besteht darin, die Anwendungsbereiche des bekannten Systems durch entsprechende Ausgestaltungen sinnvoll zu erweitern und dabei die technischen Vorzüge des Systems optimal zu nutzen.

Diese Aufgabe wird durch die Ansprüche 1 bis 15 gelöst.

Erfindungsgemäß wurde erkannt, dass das bekannte System im besonderen Maße seine technischen Vorzüge auf dem Gebiet der Medizintechnik/Ophthalmologie, insbesondere in der Präzisions-Chirurgie und auf dem Gebiet der Strabologie, d.h. bei der Untersuchung der Augenmuskulatur und der damit gekoppelten Funktiones des Auges, sowie auf dem Gebiet der Neuroophtalmologie ausspielen kann.

Dem Problem des geringen, wellenlängenabhängigen Abbildungs-(Reflexions)grads der Netzhaut kann durch geeignete Aufnahmesensoren, beispielsweise mit einer Empfindlichkeit im Bereich zwischen 0,2 und 1 mlx begegnet werden, wobei vorzugsweise eine wellenlängenabhängige Wiedergabecharakteristik Anwendung findet.

Insbesodere der in den Offenlegungsschriften DE 196 31 414 A1 und DE 197 28 890 beschriebene Scanvorgang, vorzugsweise das spiralförmige, sichtachsenidentische Scanning, erlaubt ein exaktes Wiederauffinden eines bestimmten Netzhautortes sowie eine exakte Blickrichtungsverfolgung unter Berücksichtigung der Sakkaden.

Durch die Erfindung lässt sich das System zur Aufnahme des Netzhautreflexbildes mittels eines Scansystems zum Abtasten eines Bildes auf der Netzhaut und zur Eispeisung von optischen Zusatzsignalen in das Auge durch einfache Zusatzmdule, die z.B. eine geeignete Einspeisung von Referenzinformationen in die Auswerteeinheit für die empfangenen Signale umfassen, in Geräte umwandeln, die bislang in der Medizintechnik oder Ophtalmologie als Spezialgeräte zum Einsatz gekommen sind.

Schwerpunkte der Anwendungen in der Medizintechnik liegen auf dem Gebiet der Ophthalmo-Chirurgie, der VEP (Visual Enabling for Precision Surgery), der Laser Ophthalmologie im Einsatz entsprechend einem SLO (Scanning Laser Ophthalmoloskop), der Kontrastempfindlichkeitsmessung in Abhängigkeit von der Ortsfrequenz oder der Rauschfeldampimetrie. (Mit Hilfe der auf Arbeiten von Frau Prof. Aulhorn zurückgehenden Rauschfeldkampimetrie (ein Rauschfeld entspricht z.B. einem gleichmäßigen Rauschen auf einem Fernsehbildschirm) lassen sich unter Versuchsbedingungen Gesichtsfeldausfälle mit einer Sensitivität von über 80% frühzeitig erkennen und beschreiben.

Auf dem Gebiet der Strabologie und der Neuroophthalmologie sind folgende Funktionen mit dem System darstellbar:
Die Funktion eines Koordimetrischen Geräts entsprechend dem Hess-Schirm;
Die Funktion eines Geräts zur sichtachsenidentischen Registrierung von Phorien bei unterschiedlichen Blickrichtungen;
Die Funktion eines Synoptophor/Synoptometers ohne Apparatekonvergenz;
Die Funktion eines Haploskops, insbesondere eines Phasendifferenzhaploskops, d.h. eines Geräts zur Bestimmung der relativen Breite der Konvergenz u. Fusion bei beidäugigem Sehen;
Die Funktion eines Geräts zur Bestimmung der Zyklodeviation, d.h. der Rotation des Augapfels um die Sehachse;
Die Funktion eines Geräts zur Untersuchung von Ruhestellungsfehlern, wie Winkelfehlsichtigkeiten, Phorien (Muskelungleichgewicht des Augenpaares), Tropien und Strabismus;
Die Funktion eines Geräts zur Funktionsprüfung der Netzhaut und der Analyse von Bewegungsmustern, Rauschfeldern oder des räumlichen Sehvermögens eines Auges;
Die Funktion eines Geräts zur Schielwinkelbestimmung und der Pupillomotorik.

Nachstehend werden anhand schematischer Zeichnungen Systeme beschrieben, mit welchen die erfindungsgemäßen Anwendungen in besonders vorteilhafter Weise realisierbar sind. Es zeigen:
Fig. 1 ein Schemabild eines Ausführungsbeispieles der Aufnahme- und Projektionsvorrichtung ins Auge nach Ansprüchen 16 bis 20 in einer Ausführung wo die Abbildung zwischen dem Scanner und Auge über zwei konkave reflektierende Flächen einen Hilfspiegels HS und die Innenseite der Brille BG durchgeführt wird.
Fig. 2 ein Schemabild eines Ausführungsbeispieles der Aufnahme- und Projektionsvorrichtung ins Auge nach Ansprüchen 16 bis 19 und 21 in einer Ausführung wo die Abbildung zwischen dem Scanner und dem Auge an der konkaven Hilfsspiegelfläche BG min , einer konvexen Hilfsspiegel HS min und der konkaven Innenseite des Brillenglas BG durchgeführt wird.
Fig. 3 ein Schemabild eines Ausführungsbeispieles des starren Strahlenganges zwischen Aufnahme- und Projektionsvorrichtung mit Photoempfängern und Lasermodulatoren nach Anspruch 29.
Fig. 4 ein Schemabild eines Ausführungsbeispieles der flexiblen Kopplung der Aufnahme und Projektionsvorrichtung an den Strahlumschalter und Scaneinheit mit Hilfe von flexiblen Glasfasern nach Anspruch 20.
Fig. 5 ein Schemabild eines Ausführungsbeispieles der Anbringung der Aufnahme- und Projektionsvorrichtung für beide Augen in einem Brillengestell.
Fig. 6 ein Schemabild eines Ausführungsbeispiel des Strahlenganges im Scanner bei Aufnahme des Netzhautreflexes und anschliessender Projektion des Bildes auf die Gegenstände der Aussenwelt, durch eine Umschaltung des horizontalen Scanspiegels um einen Winkel v 90 DEG nach Anspruch 38.
Fig. 7 ein Schemabild der opto-elektronischen und elektronischen Untereinheiten und ihren Verbindungen.
Fig. 8 ein Schemabild des zeitlichen Ablaufs der Abtast- und Laserprojektionsvorgänge
Fig. 9 ein Schemabild des in Mikrobauweise im Brillengestell integrierten Scanner mit Glasfaserkopplung zu einer tragbaren Empfangs- und Projektionseinheit und drahtloser Übertragung zum Bildverarbeitungscomputer;
Figur 10 eine schematische Schnittansicht des menschlichen Auges zur Erläuterung der ophthalmologischen Zusammenhänge;
Figur 11 schematisch den kozentrischen Scanvorgang bei justierten System gemäß einer Variante des Systems;
Figur 12 den Suchmode der Zentrierung des Scans durch die Augenpupille;
Figur 13 schematisch in Übersicht das ganze System; und
Figur 14 den Fluss der optischen und elektrischen sowie der Softwaresignale.

Die Erfindung arbeitet mit einer Vorrichtung, die es ermöglicht, das auf der Netzhaut des menschlichen Auges projizierte Bild der Aussenwelt in Rückstreuung aufzunehmen, dieses mit elektronischer Bildverarbeitung zu modifizieren, bzw. mit zusätzlichen Informationen zu ergänzen, und mit Hilfe einer Laserstrahlmodulation und -ablenkung zurück ins Auge mit dem Originalbild zu überlagern.

Mit dem zunehmenden Bedarf an Informationen und ihrer übersichtlichen bildhaften Visualisierung wachsen die technischen Anforderungen an die Aufnahme, Verarbeitung und Wiedergabe von Bildern. Der rasche Fortschritt auf diesen Gebieten geht Hand in mit der immer schneller werdenden Bildverarbeitung durch Computer.

Das Hauptanwendungsgebiet der elektronischen Bildverarbeitung ist heute die Weiterverarbeitung von Bildern, die von Kameras, Abtastsystemen und Sensoren, sowohl im sichtbaren Lichtbereich, als auch in and Bereichen des elektromagnetischen Spektrums, wie z. B. im Intrarot-, Radio- und Röntgenbereich, aufgenommen werden. Nach der elektronischen Bearbeitung werden die Bilder als Einzelbilder, bzw. als bewegte Bilder auf einer Bilddarstellungsfläche (Display) zur Informationsaufnahme des Aug wiedergegeben.

Mit Hilfe der elektronischen Bildverarbeitung ist es einerseits möglich, besondere Bildinhalte leichter erkennbar zu machen. Hierfür eingeführte Techniken, sind z. B. Ortsfrequenzfilterung, Kantenverschärfung, Bilddatenkompression, Bildkorrelation, Dynamikreduzierung und Falschfarbenkodierung. Andererseits befassen sich weitere Techniken mit der Überlagerung oder Subtraktion von Zusatzbildern aus verschiedenen Spektralbereichen, oder der Überlagerung von gespeicherten Plänen, Landkarten, und Zeichnungen in das Originalbild.

Für viele Anwendungen, ist eine praktisch verzögerungsfreie Bilddarstellung für das Auge von grossem Vorteil, wie z. B. beim Führen eines Flugzeuges, eines Schiffes, eines Fahrzeuges oder bei der Steuerun und Überwachung von Prozessen und Fertigungsstrassen. Mit der Bildverarbeitung kann eine gezielte Erhöhung bzw. Reduktion des Informationsgehaltes des aktuellen direkten Bildes durchgeführt werden. Die Anwendungen reichen von Erhöhung des Bildkontrastes bis zur Einblendung von Zusatzinformationen und Kennzeichnung von Details und Gefahren.

In den meisten dieser Anwendungen ist es nachteilig, dass die elektronische Kamera ein von dem Auge des Menschen getrenntes "zweites Augensystem" darstellt, denn zum einen werden die Bilder von einem anderen Aufnahmeort aus gesehen und zum anderen werden auf dem Bildschirm an einem anderen Betrachtungsort als den des Auges dargestellt. Das menschliche Auge muss somit zwischen der direkten und der indirekten Beobachtung, mit unterschiedlichen Betrachtungswinkeln, Bildausschnitten und Grössenverhältnissen ständig wechseln, was zu körperlichen Beeinträchtigungen und Verzögerungen bei Entscheidungsvorgängen führt.

Diese Einschränkung konnte zum Teil mit Hilfe der Technik des "Head-Up-Displays (HUD)", bei der Führung von Kampfflugzeugen dadurch gelöst werden, dass wichtige Informationen wie Instumentenanzeigen und Zield in die offene Brille des Pilotenhelmes und damit in das Gesichtsfeld des Piloten eingeblendet werden. Diese Technik wird auch versuchsw in der Automobilindustrie zur Einblendung von Instruinentenanzeigen in die Windschutzscheibe eingesetzt, damit der Fahrer von der Beobachtung der vor ihm liegenden Strasse nicht abgelenkt wird.

Eine bekannte Weiterentwicklung dieser Technik ist die sogenannte "virtual realitv", oder "cyberspace". Hier werden in einer geschlossenen Brille, d. h. einer Brille in der die Sicht nach aussen versperr ist, mit dem HUD bewegte räumliche Vollbilder realitätsnah in das Auge projiziert und interaktiv mit den Körperbewegungen wie etwa der Fortbewegung, Armbewegung, Fingerbewegung, Kopf- und Augenbewegung verändert.

In dem HUD wird das Bild auf einem Bildschirm generiert und nach Reflektion an der Brillenoberfläche in das Auge eingeblendet. Das Auge sieht sozusagen über die Brille als Vollspiegel auf das Display "um die Ecke herum" und bei der offenen Brille mit einem teildurchlässigen Spiegel gleichzeitig die Aussenwelt. Da das Display mit dem Kopf verbunden ist, folgt das Bild den Kopfbewegungen.

Einige HUDs sind mit einem "eye tracker" ausgerüstet, der die Augenbewegungen mit Hilfe eines Bewegungssensor am Augapfel ode einer Kamera die Bewegungen der Augenpupillen oder der Gefässstruktur der Netzhaut verfolgt. Das im HUD projizierte Bild kann dann entsprechend den Bewegungen innerhalb des Gesichtsfeldes elektronisch verschoben werden.

Zur akkommodationsfreien Entspannung des Auges kann das Bild des HUDs über die Projektionsoptik ins "Unendliche" versetzt werden. Mit der Einstellung unterschiedlicher Blickwinkel der beiden Augen z gleichen Gegenstand, wird ein stereoskopisches, d. h. räumliches Sehen ermöglicht.

Diese Anwendungen und Techniken verdeutlichen zum einen den hohen Stand der elektronischen Bildverarbeitung, die mit vertretbarem technischen Aufwand bereits in der Lage ist, bewegte Bilder mit akzeptabler Qualität fast verzögerungsfrei zu verarbeiten, zum anderen den zunehmenden Bedarf an direkter Bildübertragung ins Auge.

Der heutigen Technik der HUDs sind jedoch Grenzen gesetzt. Die Genauigkeit der automatischen Verfolgung der Augenbewegungen mit dem "eye tracker" ist wesentlich schlechter als die Ausrichtegenauigkeit und die Bildauflösung des Auges. Als Folge schwebt oder tanzt das eingeblendete Bild in dem Gesichtsfeld herum, was zu einer ungenauen Zielfindung und Ermüdung des Auges führt.

Aus diesem Grunde beschränkten sich die bisherigen Anwendungen der Vollbilddarstellung auf die geschlossene Brille, d. h. die ausschliessliche Einblendung von Fremdbildern. Die Anwendungen der offenen Brille mit der zusätzlichen Sicht nach aussen sind dagegen noch auf die Einblendung einfacher Zusatzinformationen in Form von Texten, Symbolen oder Bildumrissen beschränkt.

Eine vollständige räumliche und zeitliche Überlappung von eingeblendeten Bildern auf das von dem Auge wahrgenommene reale Bild setzt die exakte räumliche und zeitliche Übereinstimmung der beiden Bilder auf der Netzhaut voraus. Diese kann erst durch eine direkte Aufnahme des Netzhautbildes und durch eine anschliessende fast verzögerungsfreie deckungsgleiche Projektion des neuen Bildes auf das reale Bild hergestellt werden, was die Zielsetzung der Erfindung ist.

Es soll hier zunächst der Stand der Techniken der Aufnahme von Netzhautreflexbildern, der Bildabtastung aus dem Augeninneren der Projektion von Laserbildern direkt ins Auge, von der die Erfindung ausgeht, dargestellt und diskutiert werden.

Die technische Realisierung einer kontinuierlichen Abbildung des Netzhautreflexes der Aussenwelt setzt eine verwertbare optis Reflexion der Netzhaut voraus. Ihr Reflexionsvermögen ist z. B. von F.C. Delori und K.P. Pflibsen in der Arbeit "Spectral reflectance of the human ocular fundus", Applied Optics, Vol. 28, No. 6, (1989) ausführlich gemessen worden. Ab dem blauen sichtbaren Bereich (450 nm) mit dem niedrigen Wert von 0,2% ni das Reflexionsvermögen der Netzhautgrube bis 10% im langwelligen roten Bereich (750 nm) monoton zu. Im Bereich der grössten Augenempfindlichkeit und des schärfsten Sehens im grün-gelben Bereich zwischen 500 nm und 600 nm liegt das Reflexionsvermögen dann zwischen 1% und 2%.

Ein Aufnahmesystem, für diesen Reflex, muss deshalb für eine um den Faktor 50-100 geringere Leuchtdichte der Netzhaut als des Gegenstandsraumes ausgelegt sein. Eine weitere Beeinträchtigung der verfügbar Lichtmenge ist durch die Grösse der Augenpupille von 1-7 mm gegeben, die im Vergleich zu den üblichen technischen Aufnahmesystemen wie Photo- und Videokameras, relativ klein ist. Die Aufnahme des von der Netzhaut reflektierten Lichtes setzt aus diesen beiden Gründe einen besonders empfindlichen Lichtsensor voraus.

Es ist bekannt, dass ein strukturiertes Reflexbild im Bereich der Netzhautgrube oder Fovea centralis bei der Abbildung im Auge entsteht. Dies wird z. B. von Campell, F.W. and Green, D.G. in dem Artikel: "Optical and Retinal Factors Affecting Visual Resolution", J. Physiol. 181, 576-593 (1965) beschrieben. Hier wurde eine hell erleuchtete ausgedehnte Gitterstruktur auf die Netzhaut abgebildet und das durch das Auge reflektierte Bild mit einem Teilerspiegel aus dem Strahlengang abgelenkt und ausserhalb des Auges in einer Bildebene scharf abgebildet. Die flächenhafte Abbildung des Gitters nach der Reflexion an der Netzhaut, d. h. nach zweimaligem Durchgang durch das Auge, diente der Bestimmung der Modulationsübertragungsfunktion des Auges. Die photometrische Auswertung zeigte dass die Qualität des Reflexbilde der von dem Auge selbst wahrgenommene Bildqualität sehr nahe kommt.

Die von Campell u.al. verwendete geschlossene statische Aufnahmevorrichtung mit extrem heller Bildbeleuchtung (Blitzlicht) am ruhig gestellten Auge ist nicht geeignet um die lichtschwachen dynamischen Bilder der Aussenwelt auf der Netzhaut bei den raschen, natürlichen Eigenbewegungen des Auges aufzunehmen. Dies erfordert lichtempfindliche, schnelle Detektoren und eine Aufnahmetechnik, die in dem offenen Strahlengang Fremdlicht sehr effizient unterdrückt und Bilder mindestens der Wiederholfrequenz der üblichen Videonormen aufnehmen kann.

Heute kommen dafür in Frage die CCD-Kameras, die alle Bildpunkte parallel nach einer festen Integrationszeit aufnehmen, und seriell scannende Bildaufnahmesysteme mit Einzeldetektoren (Photodioden oder Photomultipliern), in denen die Bildpunkte zeitlich hintereinander abgetastet werden. Beide Techniken sind den üblichen Videonormen angepasst.

Ein grundsätzlicher Vorteil der Verwendung der CCD-Aufnahmetechnik ist die lange Integrationszeit in jedem Bildpunkt von z. B. 20 ms, gegenüber der kurzen Verweildauer in jedem Bildpunkt von nur 40 ns be Scannen. Die serielle Aufnahmetechnik hat jedoch für die Aufnahme der sehr schwachen, schnell veränderlichen Lichtsignalen bei dem stark verrauschtem Hintergrund gegenüber der parallelen Aufnahmetechnik eine Reihe anderer Vorteile, die den Nachteil der kurzen Integrationszeit wettmachen. Diese sind:
- serielle Signalverarbeitung, die eine direkte analoge Weiterverarbeitung des Bildes in Echtzeit ermöglicht - effiziente Streulichtunterdrückung durch das kleine momentane Gesichtsfeld der Abtastung - rauscharme hohe Vorverstärkung der verwendeten Avalanche-Photodioden und Photomultipliern - hohe Signaldynamik, die den starken Variationen der Bildhelligkeit auf der Netzhaut entgegenkommt - effiziente analoge Rauschunterdrückung, z. B. durch phase-lock-in- Detektion bzw. Signal-Korrelation - einfache Behebung von Abbildungsfehlern.

Für die Zielsetzung der Erfindung ist jedoch der entscheidende Vorteil der seriellen Bildabtastung die weitere Möglichkeit si mit einer zeitversetzten synchronen seriellen Laser-Bildprojektion ins Auge zu kombinieren eröffnet.

Wegen dieser Vorteile der seriellen Aufnahme gegenüber Film und Videoaufnahmen werden sie seit Anfang der fünfziger Jahre vor allem für die Bildaufnahme im Mikroskop eingesetzt. Eine serielle Abtastung kann dreierlei realisiert werden, erstens durch eine flächenhafte Beleuchtung des Gegenstandes und einer punktförmigen Abtastung mit einem Photoempfänger, zweitens der Abtastung des Gegenstandes mit einer punktförmigen Lichtquelle und flächenhafter Aufnahme mit dem Photoempfänger und drittens du die punktförmige Beleuchtung und die gleichzeitige punktförmige Abtastung mit dem Photoempfänger mit Verwendung der gleichen Scaneinrichtung. Die zwei ersten Methoden werden als "flying spot" und die dritte als "confocal scanning" Aufnahmetechnik bezeichnet.

In den beiden ersteren ist entweder die Quelle oder der Empfänger starr und dafür jeweils der Empfänger oder die Quelle auf dem Gegenstand in Bewegung. In dem dritten werden Quelle und Empfänger gemeinsam dem scannenden Abtastpunkt (konfokal) abgebildet, aber sind gegeneinander unbeweglich.

Eine Aufnahme des flächenhaften Netzhautreflexes der Aussenwelt mit einem scannendem Photoempfänger, wie die Erfindung vorschlägt, ist in diesem Sinne die erste Art der "flying spot" Bildaufnahmetechnik. Eine zeitversetzte synchrone Laserbildprojekton mit Hilfe der gleichen Scaneinrichtung kann im Sinne der gemeinsamen punktförmigen scannenden Abbildung von Beleuchtungsquelle und Photoempfänger auf der Netzhaut als eine konfokale scannende Technik aufgefasst werden, aber keine konfokal scannende Aufnahmetechnik, weil die Rollen des Photoempfängers und des Lasers gegenüber der konventionellen Anwendung vertauscht sind. In der Erfindung die die Empfangssignale zur zeitversetzten Modulation der Laserquelle, bei dem üblichen Verfahren dient die Laserquelle zur Beleucht beim gleichzeitigen Empfang der Lichtsignale.

Um den Neuheitsgrad der Erfindung und ihrer technischen Ausführungen besser sichtbar zu machen, soll der Stand der Anwendunge der Bildaufnahme und Laserprojektion ins Auge nachfolgend genauer dargelegt werden.

O. Pomerntzeff und R.H. Webb haben als erste die zweite Art der "flying spot" Aufnahmetechnik mit Hilfe eines gescannten Laserstrahles als Beleuchtungsquelle und einem starren grossflächigen Photomultiplier Empfänger zur Aufnahme der Innenstruktur des Auges in dem US Patent 4,213,678 von Sept. 1980 "Scanning Ophthahnoscope for Examining the Fundus of the Eye" beschrieben.

Eine Erweiterung dieser Technik zu einer konfokalen Anordnung mit dem gleichzeitigen scannen der Laserstrahl und der Empfangsachse des Photomultipliers, wurde von R. H. Webb, G. W. Hughes und F.C. Delori in dem Artikel "Confocal scanning laser ophthalmoscope" in Applied Optics, Vol. 26, No. 8, pp 1492-1499 (1987) vorgestellt.

In diesem Gerät wird die Netzhaut durch einen Laserstrahl rasterförmig abgetastet. Der Laserstrahl beleuchtet Punkt für Punkt und Zeile für Zeile die Vorlage. Der Photoempfänger (Photomultiplier) misst das jeweils reflektierte Licht und setzt die Messwertfolge in ein Videosignal um. Ein Fernsehmonitor stellt das Videosignal schliesslich als Bild dar. Diese drei Vorgänge erfolgen exakt synchron. Während der Laserstrahl Zeile für Zeile den Augenhintergrund abtastet, wird gleichzeitig das Fernsehsignal aufgebaut.

Der Laserstrahl durchläuft zunächst einen Modulator, über den die Beleuchtungsintensität gesteuert werden kann. Die horizonta Zeilen-Ablenkung wird meist mit einem schnell drehenden Polygonspiegel, die Vertikalablenkung durch einen Schwingspiegel durchgeführt. Der Drehpunkt der Abtastbewegung liegt in der Puppillenebene des Auges. Das vom Augenhintergrund reflektierte bzw. zurückgestreute Licht wird über die gesamte Pupillenöffnung gesammelt und dem Photoempfänger über eine Abbildungsoptik zugeleitet. Die Strahlablenkung wird dadurch rückgängig gemacht, und man erhält ein stationäres Lichtbündel, das auf einer kleinen Detektorfläch abgebildet wird.

Die Möglichkeit der Verwendung der konfokalen Abbildung im Ophthalmoskop zur Projektion von künstlichen Bildern mit Hilfe ein Laserprojektion in das Auge, wurde von Webb u. al. in dem obengenannten Artikel erkannt und wie folgt beschrieben: ... "The laser beam is deflected by a fast (15-kHz) horizontal scanner and a slow (60-Hz) vertical scan to project a standard format TV raster on the retina. Modulation of the beam permits projection of graphics or even gray scale pictures in the raster. While the patient is seeing the TV picture projected on his/her retina, an image of the retina is displayed on a TV monitor . . .".

Die Direktprojektion von modulierten Lichtreizen und Mustern wird in den modernen Laser-Scan Ophthahnoskopen (wie z. B. von der Firma Rodenstock in München) hauptsächlich für Visusanalysen, Video-Visusbestimmungen und Kontrastempfindlichkeitsmessungen bei jeweils nur einer Laserwellenlänge verwendet.

Weitere Vorschläge zur direkten Bilderübertragung mit Lasern ins Auge sind aus den folgenden zwei Schriften bekannt: In der europäischen Patentschrift 0 473 343 B1 von Nov. 1995 der Firma Sony Corporation mit dem Titel "Direct viewing picture image display apparatus" ist ein Direktsicht-Bildwiedergabegerät bekannt, das im wesentlichen nur die technischen Lösungswege aus den früher bekannten und hier bereits zitierten Veröffentlichungen der konfokalen Abbildung beinhaltet, und in den auf dem markteingeführten Laser Scanning-Ophthahnoscopen, wie der Firma Rodenstock Instrumente in München realisiert worden sind.

Die technische Lösung zur Erweiterung der Bildübertragung von nur einer auf drei Farben, die in den Ansprüchen 10 und 11 dieses Patentes beschrieben wird, ist auch in anderen Laser-Display-Anlagen seit vielen Jahren realisiert und stellt deshalb ebenso keine Neuheit dar. Die Verschiebung der Tiefenlage der Bilder auf der Netzhaut, die in den Ansprüchen 12 bis 16 beschrieben ist, wird durch gleichartige Massnahmen in den bereits existierenden Geräten durchgeführt.

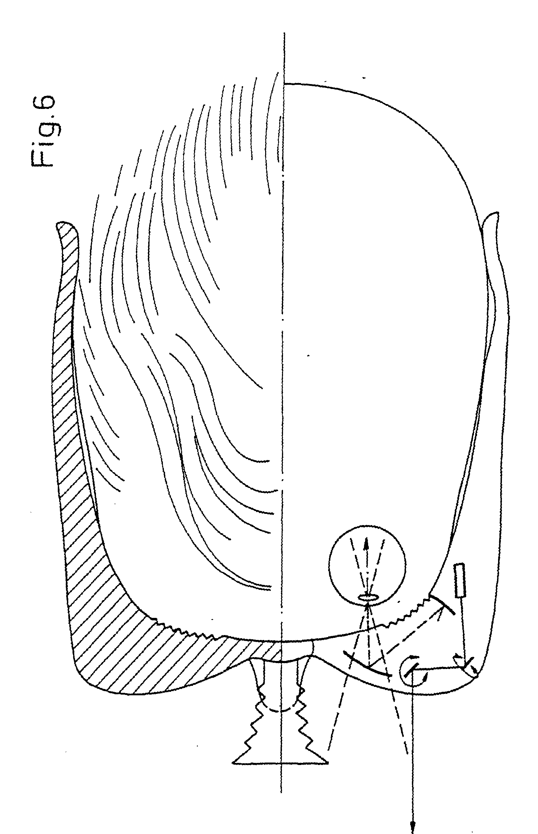

Die Trennung von zwei Strahlen durch Polarisationsunterscheidung wie sie in Anspruch 16 bis 19 und in Fig. 6 des Patentes beschrieben wird, um ein gleiches Bild in beide Augen zu projizieren, ist grundsätzlich eine ungeeignete Methode um "echte" dreidimensionale Bilder darzustellen, da diese Bilder hier keinen perspektivistischen Unterschied aufweisen. Darüber hinaus erlaubt diese Methode keine dynamische und individuelle Anpassu an die Augenausrichtung und ist deshalb technisch schwer umsetzbar.

In einer zweiten europäischen Anmeldung der Firma Motorola Inc. Nr. 0 562 742 A1 mit der Bezeichnung "Direct retinal scan display" von August 1993 wird ein Direktsicht-Bildwiedergabegerät beschrieben, das wie das oben beschriebene Patent von Sony auch die direkte Bildübertra auf die Netzhaut betrifft, jedoch mit dem Unterschied, dass die Projektion über die Umlenkung über eine dem Menschen aufgesetzte Brille durchgeführt wird.

Das System gemäß diesem Stand der Technik bringt gegenüber der lange bestehenden Technik keine neue Lösungsansätze. Die direkte Montage des gesamten Displays am Kopf des Betrachters im Anspruch 4 bzw. die Verwendung einer Umlenkung des Strahlenganges des Projektors über eine Brille im Anspruch 5 ist bei "virtualreality" Brillen bzw. bei Head-Up-Display im Pilotenhelmen bereits eingeführt.

Damit eine Abbildung auf der Netzhaut gelingt, müssen verschiedene optische Anforderungen an die Laserstrahlumlenkung erfüllt werden, die ausser der besonderen Auslegung der Strahlführung nach der Strahlablenkung, auch eine spezielle Brillenglaswölbung erfordert. Die Wege zur Lösung dieser grundlegenden optischen Problematik werden in dieser letztgenannten Patentanmeldung jedoch nicht betrachtet oder erwähnt.

Das erfindungsgemäße System schlägt eine seriell arbeitende Aufnahme- und Projektionsvorrichtung vor, die es ermöglicht, die auf der Netzha des menschlichen Auges beim natürlichen Sehvorgang erzeugten Bilder der Aussenwelt aufzunehmen und mit elektronischer Bildverarbeitung zu modifizieren, bzw. zu ergänzen. Dieses Bild dann nachfolgend mit Hilfe von Laserstrahlbildprojektion zurück ins Auge zu projizieren und mit dem Originalbild synchron zu überlagern. Die Erfindung sieht ferner vor, dass sowohl bei der Aufnahme, als auch bei der Projektion gleichzeitig die Strahlung aller Grundfarben Rot, Grün und Blau detektiert bzw. projiziert wird.

Diese Aufgabe ist grundsätzlich eine andere als die eines konfokalen Laser-Scanning-Opthahnoskops bei dem die Netzhaut im gleichen Abtastvorgang gleichzeitig beleuchtet und abgebildet wird, denn in der Anordnung gemäss der Erfindung wird in einem ersten Abtastzyklus das flächenhafte Reflexbild der Aussenwelt nach dem "flying spot" Verfahren abgetastet und erst in einem zweiten zeitlich getrennten Abtastzyklus das nachgearbeitete Laserbild auf die Netzhaut projiziert. In einem dritten Abtastvorgang wird wieder das Reflexbild gewonnen, in dem vierten wieder das Laserbild projiziert usw. Da diese Vorgänge zeitlich so schnell wechseln, entsteht für das A wie bei der Betrachtung vom Fernsehen oder Film, ein kontinuierlicher Ablauf in dem das Laserbild synchron und deckungsgleich, unabhängig von den Augenbewegungen, dem Originalbild folgt.

Die Erfindung unterscheidet sich auch von allen dem Anmelder bekannten Vorschlägen zur direkten Laserprojektion ins Auge, sowohl der Projektion von Fremdbildern in der geschlossenen Brille (cyberspace), als auch der Zusatzbildern in einer offenen Brille (HUD), durch die h zum ersten Male vorgeschlagene direkte Koppelung der Projektion mit dem momentanen Bildinhalt der Aussenwelt und die neuartigen technischen Ausführungen zu ihrer Realisierung.

Die Aufnahme und bildhafte Weiterverarbeitung des Netzhautreflexes ist durch den raschen Fortschritte in der Aufnahme- und Verarbeitungstechnik von schwachen optischen Signalen möglich geworden. Die Bestrahlungsstärke die die Netzhaut in der natürlichen Umgebung ausgesetzt ist beträgt von der hellsten Aussenbeleuchtung von 10<-4> W cm<-2> bis etwa 10<-7> W cm<-2>. Bei schwacher Innenbeleuchtung, unter Lesebedingungen beträgt sie 10<-5> W cm<-2> bis 10<-6> W cm<-2> (siehe z. B. "Safety with Lasers and Other Optical Sources", D. Sliney and M. Wolbarsht, 1980). Mit einem Photon-Counting Photomultiplier und punktueller Abtastung mit Lasern in einer TV-Norm wurde eine Empfindlichkeit bis zu 2 x 10<-5> W cm<-2> bei einem Signal-Rauschverhältnis von 5 erreicht (s. R.H. Webb et al., "Flying spot TV ophthalmoscope", Applied Optics, Vol. 19, No. 17, pp. 299 (1980).

Eine Erhöhung der Empfindlichkeit bis 10<-7> W cm<-2> kann z. B. erreicht werden durch verbesserte Rauschunterdrückung, eine verringerte Ortsauflösung, bzw. durch die Verwendung eines Spiral-Scans anstatt des TV-Rasterscans, der eine reduzierte ScanGeschwindigkeit in der Mitte des Gesichtsfeldes und damit eine verlängerte Integrationszeit dort bietet.

Die Aufgabe der Erfindung wird durch die in Anspruch 1 und 2 angegebenen Merkmale gelöst. In den Unteransprüchen sind vorteilhafte Ausgestaltungen und Weiterbildungen angegeben.

In der nachfolgenden Beschreibung wird das System, mit dem die ophthalmologischen/medizinischen Anwendungen in vorteilhafter Weise ausgeführt werden können anhand von Ausführungsbeispielen erläutert. Die Figuren ergänzen diese Erläuterungen.

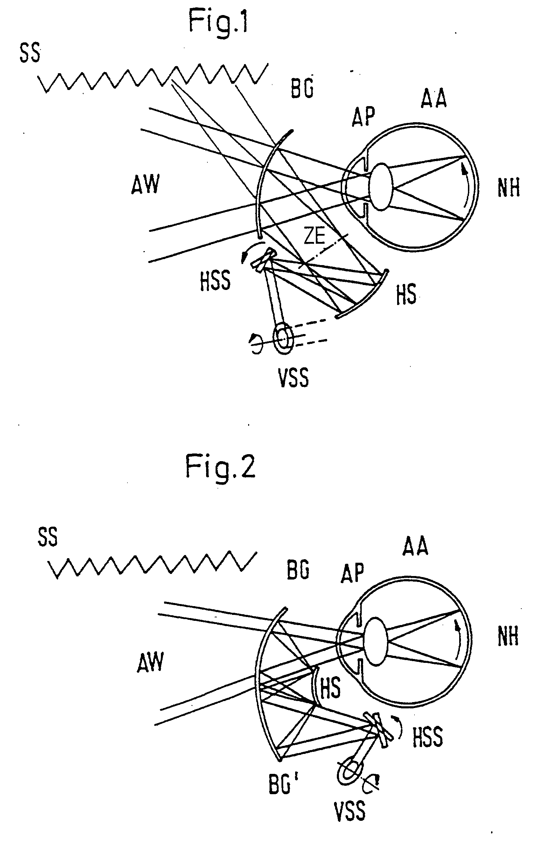

Die Erfindung schlägt gemäss dem Anspruch 16 eine scannende Aufnahmevorrichtung vor, um den lichtschwachen Reflex von Gegenständen der Aussenwelt AW auf der Netzhaut NH des Auges AA seriell aufzunehmen wie, in den Fig. 1 bis Fig. 3 dargestellt ist.

Die gleiche Abbildungs- und. Scanvorrichtung wird auch verwendet um das verarbeitete Bild mit Hilfe von Lasern und Bildmodulatoren über den entgegengesetzten Lichtweg auf die Netzhaut zeitversetzt gemäss Anspruch 2 zu projizieren, wie auch in Fig. 1 bis Fig. 3 dargestellt ist.

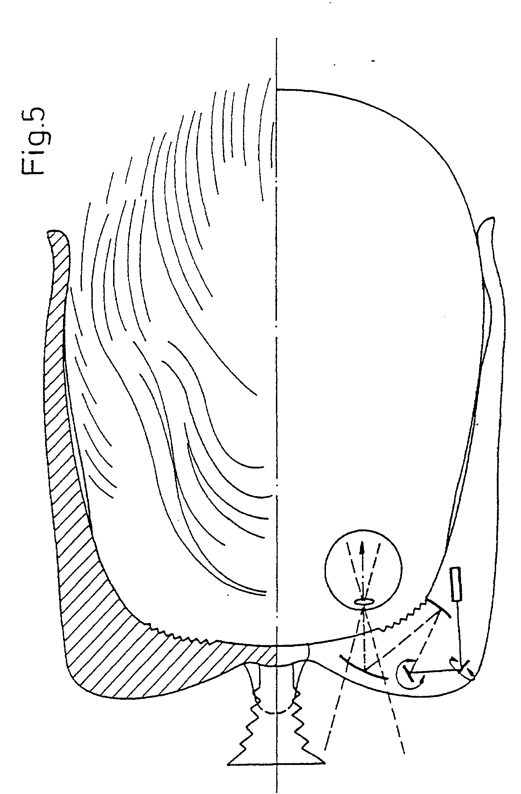

Der zentrale Gegenstand der Erfindung ist eine spezielle Brille, die einem Betrachter aufgesetzt wird, wie in Fig. 5, Fig. 6 und Fig. 9 gezeigt ist. Die Brillengläser BG dienen als Strahlteiler. In Transmission, für das Licht aus der Aussenwelt und in Reflexion, als eine Abbildungsfläche des von der Netzhaut durch das Auge rückgestreuten Lichtes, das mit Hilfe weiterer Abbildungselemente und eines zweiachsigen Scanner für die Horizont HSS und Vertikalablenkung VSS einem Photoempfänger zugeführt wird (Fig. 1 bis Fig. 4).

Der Strahlengang wird gleichzeitig derart gestaltet dass die Verlängerung der Sichtlinie vom Detektor durch die Brille immer in der absorbierenden Schicht einer Strahlungssenke SS mündet. Die Verlängerung der Sichtlinie des Auges durch die Brille läuft dagegen zur Aussenwelt AW (Fig. 1 bis Fig. 6).

Die einfachste Methode der Strahlteilung an den Brillengläsern BG ist die Verwendung von 50% transmittierenden und 50% reflektierenden Spiegelgläsern. Es können auch aktive elektronisch steuerbare Spieg die von vollständiger Transmission bis vollständiger Reflexion in den beiden Abtastzyklen umschalten, eingesetzt werden.

Das Auge AA bildet von der Aussenwelt AW parallele oder nahezu parallele Strahlbündel auf der Netzhaut ab. Der Drehpunkt der Strahlbündel bei unterschiedlichen Betrachtungswinkel zur Aussenwelt liegt i der Pupille des Auges AP.

Die Erfindung geht von einer gleichzeitigen Aufnahme und Projektion in beide Augen wie in Fig. 5 und Fig. 6 gezeigt wird und damit einem weitgehend identischen Strahlengang für das linke und rechte Auge. Bei Fehlsichtigen, auch mit unterschiedlicher Brechkraft des linken und rechten Auges, sieht die Erfindung vor, dass entweder die Brillengläser in ihrer Brechung durch entsprechende unterschiedliche Auslegung der Wölbung der Aussenseit und der Innenseite individuell angepasst sind, bzw. dass Kontaktlinsen getragen werden. Für Normalsichtige ist die Wölbung der Innen und Aussenseite der Brillengläser die Brillengläser BG identisch.

Das von jedem einzelnen Bildpunkt der Netzhaut aus dem Auge zurrückgestreute Licht ist in gleicher Weise eine nahezu parallel Strahlenbündel, der den identischen Weg wie das einfallende Licht in entgegengesetzter Richtung auf die Innenseite des teilweise reflektierenden Brillenglases BG fällt. Die Krümmung dieser Fläche wird so gestaltet, dass gemeinsam mit der Augenlinse ein zweites Bild des Bildpunktes auf der Netzhaut in der Zwischenebene ZE entsteht (Fig. 1). Ein Hilfsspiegel HS, kollimiert die Strahlen erneut und bildet sie so ab, dass sie über den gemeinsamen Drehpunkt (sie auf der anderen Seite durch die Augenpupille) auf der Achse des horizontalen Scannerspiegels HSS verläuft. Eine vertikale Ablenkung wird durch einen zweiten Scannerspiegel VSS durchgeführt.

Die Abbildung aus und ins Auge mit Hilfe der beiden Spiegel, Hilfsspiegel und Brillenglasspiegel bei gleichzeitiger freier Sicht durch das Brillenglas BG zur Aussenwelt AW erfordert relativ starke Strahlablenkung. Die gegenläufige Umlenkung über zwei konkave Spiegelfläche hebt zum Teil dabei auftretenden Abbildungsfehler auf. Der gegenläufige sonst identische Strahlengang von Bildaufnahme und Bildprojektion vermeidet auch zum grossen Teil die Entstehung von Bildverzerrungen im Auge auf.

Bei sphärischen Spiegeln treten jedoch wegen ihren starken Abbildungsfehlern trotzt der relativ kleinen erforderlichen Ablenkwinkel von < +/- 10 DEG restliche Bildstörungen auf. Für die Abbildung und Umlenkung ins Auge sind deshalb hochwertigere Spiegelsysteme, wie konkave Parabolspiegel und elliptische Spiegel in Anspruch 5 vorgesehen. Eine effiziente Reduktion der Abbildungsfehler ist nach Anspruch 6 auch mit Hilfe der Spiegelu an zwei konkaven BG und BG min und einer konvexen Fläche HS möglich. Hier kann als Vollspiegelfläche BG min die zweite Hälfte des Brillenglases gleicher konkaven Krümmung wie BG verwendete werden.

Die Erfindung geht davon aus dass jede Art von zweiachsigen Bildscannern verwendet können wie z. B. Drehspiegel oder Polygonspiegel für Zeilenablenkung und Schwingspiegel für Vertikalablenkung bzw. akustooptische Ablenkeinheiten für beide Achsen.

Mit einer rasterförmigen Abtastspur mit getrennter horizontaler und vertikaler Ablenkung kann der Aufbau des Bildes konform mit den gebräuchlichen Video-Normen wie VHS und NTSC und HDTV gestaltet werden (Anspruch 24).

Es können aber andere Abtastspuren, die dem Bildaufbau des Auges besser angepasst sind als der Rasterscan, verwendet werden, wie z. B. ein Spiralscan. Die grösste Sehscharfe in der Netzhaut (Retina) ist im Bereich der sogenannten Fovea centrales, die im Gesichtsfeld nur einen kleinen Winkelbereich von etwa +/- 2 DEG um die Sehachse aufnimmt. Wenn sich die Aufmerksamkeit auf ein Objekt richtet, werden die Augen normalerweise so bewe dass die vom fixierten Objekt ausgehenden Strahlen auf die Fovea centralis fallen.

Ein Spiralscan der Bildabtastung, in dem die Aufenthaltsdauer des Abtaststrahles in Richtung zur Sehachse kontinuierlich zunimmt, wäre dadurch wesentlich besser dem Aufbau der Netzhaut angepasst als ein Rasterscan. Durch die verlängerte Aufenthaltsdauer wird dadurch auch ein entsprechend höheres Signal/Rauschen-Verhältnis in dem mittleren Bereich erreicht.

Die Erfindung sieht aus diesen Gründen ausser der Verwendung eines Rasterscans in Anspruch 24 auch in dem Anspruch 25 auch vor dass durch eine entsprechende Auslegung und Ansteuerung der beiden Strahlablenkeinheiten ein Spiralscan auch verwendet werden kann.

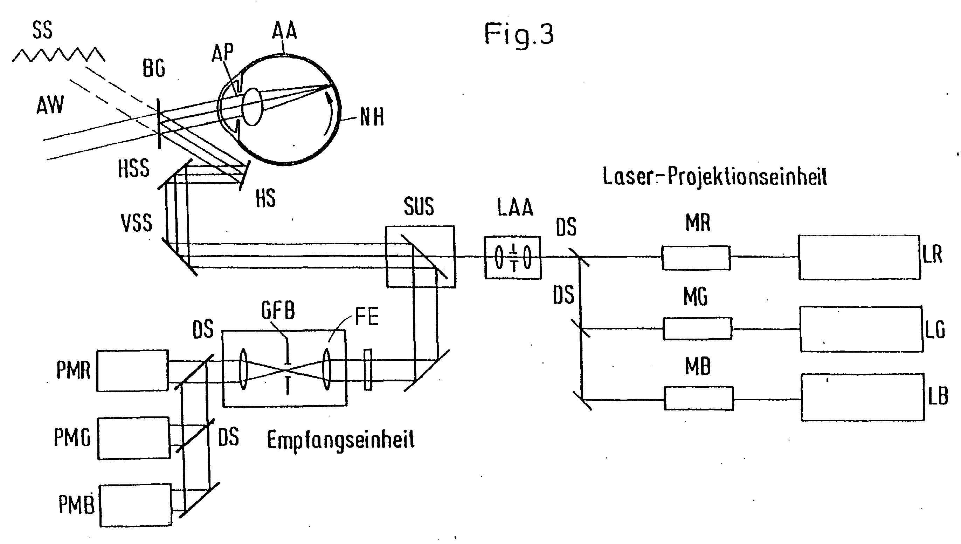

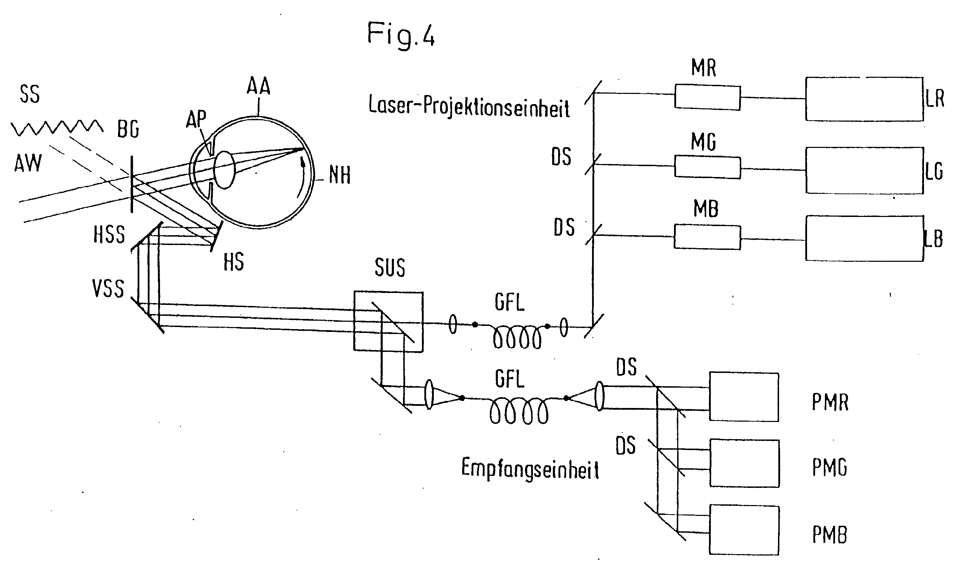

Wie in einem Laser-scanning-ophthalmoskop wird der Strahlengang zwischen dem Projektions- und Empfangskanal mit Hilfe eines Schaltspiegels SUS getrennt. Da der Durchmesser des Projektionsstrahls wegen guten Bündelung und dem kleinen Durchmesser der Laserstrahlen kleiner ausgelegt werden kann als der Empfangsstrahl bietet sich die Möglichkeit an einen Lochspiegel zur Trennung der beiden Strahlengänge wie in Fig. 3 und Fig. 4 dargestellt ist, zu verwenden. Eine effizientere Methode, die sich wegen der zeitlich alternierenden Verwendung der beiden Strahlengänge ergibt, der Einsatz eines Kippspiegels der die Strahlengänge synchron mit der Abtastung umschaltet. Diese Lösung hat die Vorteile der geringeren optischen Verluste im Empfangskanal und der besseren optischen Abschirmung des dire Übersprechens von Projektions- in den Empfangskanal.

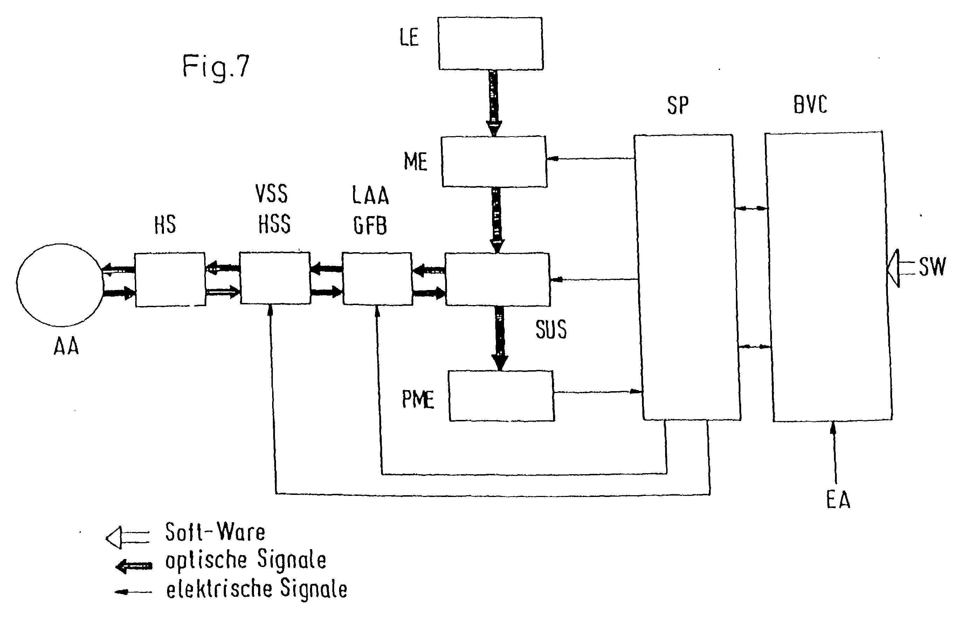

Im Strahlgang der Projektionseinheit hinter dem Strahlschalter SUS ist wie in Fig. 3 gezeigt sieht die Erfindungsmeldung im Anspruch 32 eine Fokussiereinrichtung FE, die die Grösse des Laserbildflecks in Anspruch 32 und des abgetasteten Flecks beim Empfang GFB in Fig. 3 auf der Netzhaut einstellt. Zur Einstellung des von den Photomultipliern gesehenen momentanen Gesichtsfeldes dient nach Anspruch 33 eine gemeinsame Gesichtsfeldblende GFB im Strahlengang zweier Linsen. Die Einstellung der Gesichtsfeldblenden ist zur Anpassung an die Beleuchtungsverhältnisse an der Netzhaut und zur Einstellung der erwünschten Ortsauflösung notwendig. Es ist vorgesehen, dass beide Einstellung über Aktoren automatisch nach Rechnerbefehl durchgeführt werden können wie in Fig. 7 dargestellt wird.

Es ist in Anspruch 27 vorgesehen, dass der Netzhautreflex mit der Verwendung von dichroitischen Filtern (DFR, DFG und DFB) und drei getrennten Detektoren (PMR, PMG und PMB) in bis zu drei Farbkanälen aufgeteilt ist und damit ein weitgehend unverfälschtes Farbbild aufgenommen werden kann. Auf der Laserseite wird ebenso mit dichroitischen Strahlteilern die Strahlen von bis zu drei Lasern im roten, grünen und blauen Spektralbereich (LR, LG, LB) nach der getrennten Bildmodulation jeder Farbe (MR, MG, MB) auf einer gemeinsamen Achse vereinigt.

Zur farbtreuen Bildaufnahme wird das optische Signal mit dichroitischen Filtern DFR DFG und DKB im Empfangskanal in die drei Farbkomponenten vor den drei Photoempfängern, verzugsweise Photomultipliern PMR, PMG und PMB in die drei Grundfarben zerlegt und getrennt vermessen. Wegen der schwachen Lichtsignale werden vor allem Photon-counting Verfahren zum Einsatz kommen.

Die Erfindung sieht ferner vor, dass das von dem Detektor aufgenommene elektronische Bild nach einer Bildverarbeitung mit Hilfe von Laserstrahlquellen und -modulatoren wieder in ein serielles optisch Bild umgewandelt wird, und in einem zweiten Bildzyklus mit der gleichen optischen Einrichtung, - jetzt in der Funktion als Strahlablenkeinheit (Laser-Scanner) - nach Reflexion an der Brillenglasinnenfläche in das Auge synchron, aber zeitversetzt mit der Abtastung des Originalbildes zurückprojiziert wird.

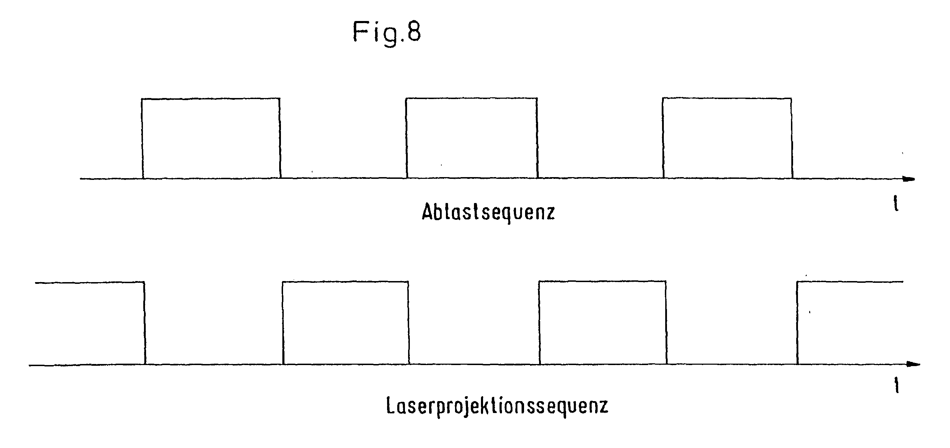

Die Erfindung schlägt vor in Anspruch 35 die Perioden der Bildaufnahme und Bildprojektion zeitlich zu trennen, d. h. alternierend durchzuführen wie in Fig. 8 dargestellt ist, um eine Störung der Aufnahme des schwachen Netzhautbildes der Aussenwelt durch die lichtstärkere Projektion zu vermeiden. In einem ersten Bildzyklus wird z. B. das Netzhautreflexbild aufgenommen und in dem zweiten wird das verarbeitete elektronische Bild in das Auge projiziert. In dem dritten Bildzyklus wird eine Aufnahme des Netzhautreflexbildes durchgeführt, in dem vierten wird wieder die Zurückprojektion durchgeführt, usw.

Wenn dieser Bildwechsel schnell genug ist, sorgt die Trägheit der Gesichtsinnes dafür, dass beide Bilder dem Betrachter überlagert erscheinen, vorausgesetzt, dass die Zeitverzögerung für das in das Auge eingeblendete Bild unterhalb der Bewegungsdauer und Wahrnehmungszeit des Auges liegt, und dass die Stabilität und Auflösung des eingeblendeten Bildes mit der Auflösung des Auges vergleichbar ist.

Damit, sowohl die unbewussten schnellen Sakkadenbewegungen des Auges mit einer mittleren Amplitude von 5 Bogenminuten und einer Dauer zwischen 10 und 20 msec, als auch die schnellen Augenbewegungen von 20 DEG -30 DEG /sec beim Verfolgen eines bewegten Objektes über einen grösseren Winkel erfasst werden können, muss die Bildwiederholfrequenz ausreichend hoch sein. Mit einer Wiederholfrequenz zwischen 50 Hz bis 100 Hz, sowie in de Fernsehen- und Computertechnik, ist die Aufnahme den schnellsten Bewegungsvorgängen des Auges weitgehend angepasst. Dies gilt, sowohl für einen Raster- als auch einen Spiralscan.

Weitere technische Anforderungen an die Aufnahmevorrichtung betreffen die Grösse des erfassten Gesichtsfeldes und die Bildauflösung der hier vorgeschlagenen Vorrichtung. Für die meisten Anwendungen ist Bereich des schärfsten Sehens mit einem Durchmesser von 10 und einer Anzahl von 7 Millionen Zapfen (Bildpixel) in der Netzhautgrube (Fovea) aber auch der angrenzende Bereich mit wesentlich geringerer Auflösung bis etwa 10 DEG Durchmesser von Interesse. Für diese Unterschiedlichen Auflösungsanforderungen ist gerade der Spiralscan der Abtastspur besonders geeignet.

Als Lichtquellen zur Rückprojektion der Bilder ins Auge sind Halbleiterlaser bzw. miniaturisierte Festkörperlaser vorgesehen mit einer niedriger Dauerstrichleistung (< 300 mu W), die keine Gefährdung des Auges verursachen können. Mit der Verwendung von Halbleiterlasern könnte die Bildmodulation direkt über ihre Stromversorgung durchgeführt werden. Damit alle Farben erzeugt werden empfiehlt sich die Verwendung von drei Lasern mit den Grundfarben rot, grü blau. Wie das bekannte Farbdreieck des menschlichen Gesichtsinnes zeigt können alle anderen Farben sowie die Unfarben grau und weiss durch Farbsummation von monochromatischen Laserlinien dieser Farben gebildet werden. Die Erfindung beinhaltet auch die Möglichkeit der Verwendung von einzelnen Farben als monochromatische Lösung.

Die Erfindung sieht wie in Fig. 7 dargestellt einen Signal-Prozessor SP vor, der das direkte Bild von der Netzhaut elektronisch bearbeitet und alle Funktionen der Vorrichtung sowie die von Scannern VSS/HSS und Laserfleckeinstellung und Grösse der Gesichtsfeldblende LAA/GFB synchron koordiniert. Der Bildverarbeitungscomputer BVC übernimmt dann die vom Auge wahrgenommenen Bild oder Bilder anderer technischer Sensoren die über einen externen Anschluss EA dem Computer zugeführt werden und bearbeitet nach einer vorgegebenen Software SW, bevor sie mit Hilfe des Signalprozessors auf die Laserstrahlen als Bildsignal aufmoduliert werden.

Die Laser-Projektion ermöglicht ausser der Verarbeitung des aktuellen vom Computer verarbeitetes Bild in das Auge zu projizieren und mit dem Originalbild zu verschmelzen, auch Fremdbilder, die dem Compu von extern zugeleitet werden, dem Aussenbild im Auge synchron zu überlagern. Wenn die Zeitspanne zwischen Bildaufnahme und -Projektion im Vergleich zu den schnellen Augenbewegungen entsprechend kurz ist, wird das Auge, wie bei Betrachtung eines Fernsehschirmes, keine Bildunterbrechung mehr wahrnehmen.

Die getrennte aber gleichzeitige Bildabtastung an beiden Augen erfasst auch die perspektivischen Unterschiede beider Bilder. Da diese bei der LaserZurückprojektion in beiden Augen erhalten bleiben, ist eine Wiederherstellung des räumlichen Sehens gewährleistet.

Ausser der Projektion von den Netzhautbildern nach Bildverarbeitung zurück ins Auge ermöglicht die hier vorgeschlagene Einrichtung nach Anspruch 40 auch die Projektion dieser Laserbilder direkt auf die in der Umwelt befindlichen und vom Auge gesehenen Gegenstände. Dies Ausfüh der Erfindung wird schematisch in Fig. 6 durch das Umklappen des Scanspiegels um einen Winkel von 90 DEG schematisch dargestellt.

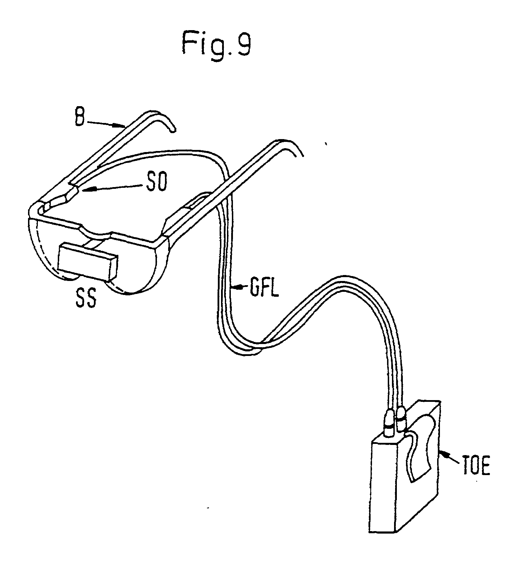

Die in der Erfindung verwendeten Bauelemente sind heute weitgehend miniaturisiert und kostengünstig erhältlich. Die Strahlumlenkeinheit und Scanner können in einem einfachen Brillengestell B wie in Fig. 9 dargestellt ist untergebracht werden. Mit Hilfe von Glasfaserleitung GFL können Laserprojektionseinheit und Empfangseinheit abgesetzt in einem kleinen Gehäuse TOE, z. B. der Grösse eines Taschenbuches mit Batterieversorgung untergebracht werden. Der Datenaustausch mit einem externen fest installierten Bildverarbeitungsrechner kann entweder über Radiowellen oder Infrarotstrahler erfolgen. Alle Elemente der Vorrichtung der Erfindung könnten nach dem heutigen Stand der Technik somit von einem Menschen mühelos getragen werden und der drahtlose Bilddatenaustausch mit dem externen Rechner würde sei unbeschränkte Bewegungsfreiheit ermöglichen.

Das vorstehend beschriebene System hat neben den erfindungsgemäßen Anwendungen auf dem Gebiet der Medizintechnik/Ophthalmologie und der Strabologie/Neuroophthalmologie noch eine Reihe verschiedenster Einsatzbereiche, auf die an dieser Stelle nicht erneut eingegangen werden soll. Sie sind in der Patentanmeldung DE 196 31 414 ausführlich beschrieben und können in den nachfolgenden vier Kategorien zusammengefasst werden:
- Aufnahme von Bildern der Aussenwelt, ihre Verarbeitung, Zurückprojektion und Verschmelzung mit dem Originalbild im Auge. - Überlagerung von Bildern anderer Aufnahmesysteme, z. B. in von der gleichen Szene in anderen Spektralbereichen auf das direkte Bild. - Überlagerung von virtuellen Bildern, die alleine vom Computer hergestellt werden und - Aufnahme von Bildern der Aussenwelt und ihre Laser-Projektion nicht ins Auge, sondern auf die gleichen, vom Auge gesehenen Gegenstände der Aussenwelt.

In der ersten Kategorie sind Anwendungen mit dem Ziel, das von dem Auge aufgenommene Bild durch gezielte Bildsummation zu verbessern, z. B. ein verschwommenes oder lichtschwaches Bild zu verschärfen u verstärken, was für Sehbehinderte, aber auch für Normalsichtige von grosser Hilfe wäre.

Andere mögliche Bildänderungen wären z. B. die Änderung der Farbe von Objekten durch neue Farbsummation. Diese Technik könnte zur gezielten Weissfärbung von bestimmten Bereichen des Sehfeldes, und damit zur Auslöschung oder Verminderung der optischen Informationen verwendet werden.

Die zweite Kategorie beinhaltet Überlagerung von Bildern der gleichen Szene z. B. aus dem unsichtbaren intraroten Bereich oder von Radargeräten. Diese Technik würde z. B. das Fahren oder Fliegen in der Nacht und bei Nebel oder Dunst wesentlich erleichtern.

In medizinischen Anwendungen könnten z. B. Röntgenbilder, akustische Bilder und Bilder vom Kernspintomographen dem direkten Bild des Körpers des Patentien oder seiner Organe zur Hilfestellung des Arztes b der Diagnose und in der Chirurgie überlagert werden.

Die dritte Kategorie umfasst Anwendungen, in denen das Bild durch virtuelle Zusatzeinblendungen ergänzt werden, wie z. B. in den Anwendungen der heutigen HUDs bei dem Führen von Fahrzeugen. Die Erfindung bietet den zusätzlichen Vorteil der exakten Synchronisation der Einblendung mit dem Aussenbild. Dadurch könnten Fremdbilder auf genau definierten freien Stellen innerhalb des Direktbildes, z. B. mit geringem Bildinhalt, oder als Stereobild in einer anderen Entfernung als die übrigen Gegenstände, eingeblendet werden.

Zu dieser dritten Kategorie gehören interaktive Anwendungen aus der Computertechnik, d. h. die Einblendung einer virtuellen Computermaus (Zielkreuzes), die alleine mit den Augenbewegungen (anstatt mit der Hand) über reale Gegenstände der Aussenwelt (auch Display) geführt werden. Anklicken, oder ein Befehl könnte hier durch zusätzliche Augenbewegungen z. B. Augenlidschlag, oder Stimme oder mit Tastendruck ausgeführt werden.

Diese dritte Kategorie beinhaltet auch cyberspace-Anwendungen, d. h. die Einblendung von virtuellen Computervollbildern in die geschlossenen Brillen. Mit Hilfe der Erfindung könnten Aufnahmen des Netzhautbildes der eingeblendeten virtuellen Bilder genutzt werden, um diese gegen den Augenbewegungen zu stabilisieren.

Die vierte Kategorie beschreibt eine Art "aktives Sehen", d. h. eine vom Auge gesehene und von der Abtastvorrichtung aufgenommene Szene wird in dem nächsten Abtastzyklus mit einem Laser-Bildscheinwerfer seriell beleuchtet. Diese so beleuchtete Szene wird wieder vom Auge wahrgenommen und führt in dem darauffolgenden Zyklus zu einem verändertem zweiten Laser- Beleuchtungsvorgang, der von einem dritten Vorgang, usw. gefolgt wird.

In dieser Weise entsteht eine optische Rückkoppelungsschleife, die durch eine entsprechenden Auslegung der Beleuchtung als positive oder negative Rückkoppelung für die verschiedensten Anwendungen genutzt we kann, z. B. um schwach erkennbare Objekte zu erhellen, deren Kontrast zu erhöhen oder ihre Farbe zu ändern.

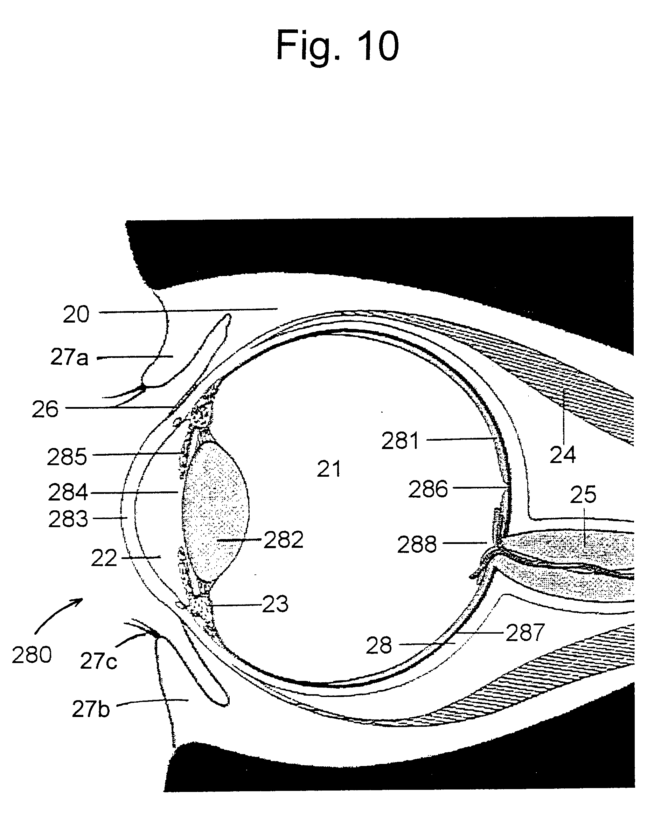

Figur 10 zeigt zwecks besserem Verständnis der Erfindung eine detaillierte Ansicht eines Auges 280 im Querschnitt. Das Auge 280, das in einer aus Schädelknochen gebildeten Augenhöhle 20 (lat. Orbita) im Kopf eines Menschen untergebracht und hier im Sinne eines Augapfels 280 zu verstehen ist, besteht aus einer von einer lichtdurchlässigen Hornhaut 283 (lat. Kornea) und einer sichtlich weißen Lederhaut 28 (lat. Sklera) umgebenen Kammer. Die Lederhaut 28 ist auf seiner dem Inneren des Auges 280 zugewandten Seite von einer Aderhaut 287 (lat. Choroidea) überzogen, die auf seiner ebenfalls inneren Seite eine lichtempfindliche Netzhaut 281 (lat. Retina) trägt und diese mit Blut versorgt. Durch ihre Pigmentierung verhindert die Aderhaut 287 eine Steuung des darauffallenden Lichts, die das Sehvermögen stören könnte.

Das Gewebe der Netzhaut 281 umfaßt zwei Arten von Photorezeptorzellen, nämlich Stäbchen und Zapfen (beide nicht dargestellt), die dem Menschen den Sehsinn ermöglichen. Diese Photorezeptorzellen absorbieren das durch eine Augenlinse 282 gebündelte Licht in einem Wellenlängenbereich von ca. 380-760 nm und verwandeln es durch eine Reihe von chemischen Reaktionen in elektrische Nervensignale. Die Signale der verschiedenen Nervenzellen der Netzhaut 281 werden dann über einen Sehnerv 25 an das Gehirn weitergeleitet und dort zu einem wahrnehmbaren Bild verarbeitet. Die zahlreichen, ca. 120 Millionen zählenden und stark lichtempfindlichen Stäbchen sind auf die Signalaufnahme im Dämmerlicht spezialisiert und liefern ein Graustufenbild. Die ca. 6,5 Millionen, vergleichsweise weniger lichtempfindlichen Zapfen dagegen sind für die Verarbeitung bei Tageslicht und für das Farbensehen zuständig. Bei der Lichtabsorbtion findet eine Oxidierung von Pigmenten in den Photorezeptorenzellen statt. Zur Regenerierung der Pigmente bedarf es bei den Zapfen ca. 6 Minuten und bei den Stäbchen ca. 30 Minuten. Eine Betrachtungsdauer von ca. 200 msec ist notwendig, bis der Sehreiz über die Photorezeptoren einsetzt und eine Informationsaufnahme über die Netzhaut 281 erfolgt.

Die Netzhaut 281 weist eine Vertiefung 286 auf, die durch ihre im Vergleich zur übrigen Netzhaut höher Dichte an Zapfen als etwas stärker pigmentiert erscheint. Diese Vertiefung 286, die üblicherweise Sehgrube 286 (Fovea centralis) genannt wird, liegt in einem als "gelber Fleck" (lat. Makula) bekannten Bereich der Netzhaut und stellt den Bereich des schärfsten Sehens dar. Die Fovea centralis 286 ist nur mit Zapfen besetzt, weist eine sehr hohe Zapfendichte auf und beansprucht lediglich ca. 0,01% der Netzhautoberfläche. An der mit dem Bezugszeichen 288 gekennzeichneten Stelle vis-à-vis der Linse 282 tritt das Sehnerv 25 durch eine siebartige Öffnung in der Lederhaut 28 in das Innere des Auges ein. Diese Stelle 288 weist keine Photorezeptorzellen auf, weshalb sie als "blinder Fleck" bezeichnet wird.

Die von der Hornhaut 283 und der Lederhaut 28 gebildeten Kammer ist durch eine verformbare Linse 282 und einen muskelösen Strahlenkörper 23 (auch Ziliarkörper genannt), der die Linse 282 trägt, unterteilt. Der zwischen der Linse 282 und der Netzhaut 281 liegende Teil der Kammer, der ca. 2/3 des Augapfels ausmacht, bildet einen sogenannten Glaskörper 21, ein gallertiges Gebilde, das zu über 98% aus Wasser besteht und die Netzhaut 281 stützt und schützt. Der als Vorderkammer 22 bezeichnete, zwischen der Hornhaut 283 und der Linse 282 liegende Teil der Kammer enthält eine Flüssigkeit, die die Hornhaut 283 ernährt. In ihrer Urform bricht die Linse 282 das auf das Auge fallende Licht typischerweise derart, daß das ferne Sehfeld auf die Netzhaut 281 scharf abgebildet wird. Durch Anspannung/Entspannung der Muskeln des Ziliarkörper 23 kann die Form und somit auch die Brechungscharakteristik der Linse 282 über einen breiten Bereich verändert werden, um beispielsweise eine scharfe Abbildung nahliegender Gegenstände des Sehfelds auf die Netzhaut 281 zu ermöglichen. Dieser Vorgang läuft in den meisten Fällen für den betroffenen Menschen unbewußt ab.

Unmittelbar vor der Linse 282 befindet sich in der Vorderkammer 22 eine aus gefärbtem Gewebe bestehende Blende 285 veränderbaren Durchmessers, die den Lichteinfall auf die lichtempfindlichen Teile des Auges 280 reguliert und dem Auge 280 seine charakteristische Färbung verleiht. Diese Blende 285 wird deshalb als Regenbogenhaut 285 (lat. Iris) bezeichnet. Aufgrund der geringen Lichtrückstrahlung der Linse 282, des Glaskörpers 21 und der Netzhaut 281 erscheint der zentrale Bereich der Iris 285 schwarz und wird Pupille 284 bezeichnet. Auch die Regulierung der Pupillengröße läuft für den Menschen unbewußt ab.

Das Auge 280 ist über sechs teils parallel, teils schräg zueinander verlaufende Muskeln 24 an die Schädel verbunden, die ein Schwenken des Auges 280 und folglich eine Änderung der Blickrichtung ermöglichen. Das binokular, ohne Bewegung der Augen 280 erfaßte Gesichtsfeld umfaßt horizontal ca. 170° und vertikal ca. 110°. Werden die Augen 280 bewegt, kann ein binokulares Blickfeld von horizontal ca. 290° und vertikal ca. 190° erfaßt werden. Der von der Fovea centralis 286 erfaßten Bereich des schärften Sehens umfaßt lediglich ca. 1°. Eine fiktive Achse durch die Mitte dieses Bereichs wird als Sehachse bezeichnet und entspricht der Blickrichtung. Auch eine Rotation des Auges um die Sehachse wird durch die Muskeln 24 ermöglicht.

Die sechs Muskeln 24 sind für sämtliche Augenbewegungen zuständig. Bei einer Betrachtung eines Fixpunkts finden sogenannte Mikrotremors des Auges 280 statt, bei denen das Augen 280 leicht zittert, um eine vorübergehende Erschöpfung der chemischen Reaktionfähigkeit der betroffenen Photorezeptorzellen beim gleichbleibenden Reiz zu vermeiden. Während eines Blickrichtungswechsels oder einer Kopfbewegung finden sogenannte Sakkadenbewegungen statt, mit deren Hilfe die Fovea centralis 286 auf ihr neues Fixationsziel gerichtet bzw. auf ihr bisheriges Fixationsziel gehalten wird. Bei dieser sehr komplex ablaufenden Bewegung wird das Auge 280 unwillentlich mit einer kleinen Amplitude von bis zu mehreren zehn Grad und einer extrem schnellen Winkelgeschwindigkeit von bis zu mehreren hundert Grad pro Sekunde hin und her bewegt. Bei der Verfolgung eines sich bewegenden Objekts erreicht das Auge 280 Winkelgeschwindigkeiten von lediglich eins bis zwei hundert Grad pro Sekunden.

Zum Schutz des Augapfels 280 hat der Mensch bewegliche Hautfalten, nämlich ein Oberlid 27a und ein Unterlid 27b, die ein Schließen der Augenhöhle 20 gegen äußere Einflüsse ermöglicht. Die Lider 27a und 27b schließen sich reflektorisch bei einfallenden Fremdkörpern und starker Blendung. Darüber hinaus sorgen die Lider 27a und 27b durch regelmäßigen, meist unwillkürlichen Lidschlag für einen gleichmäßig auf der Hornhaut 283 verteilten Tränenfilm, der die äußere Oberfläche der Hornhaut 283 vor einem Austrocknen wahrt und wäscht. Die Lider 27a und 27b weisen auch Wimpern 27c auf, die das Auge 280 ebenfalls vor Staub schützen. Eine Bindehaut 26 kleidet den Raum zwischen den Lidern 27a bzw. 27b, der Aughöhle 20 und dem Augapfel 280 aus. Die Bindehaut 26 geht einerseits in die Lidinnenseite über,-andererseits in die Hornhaut 283, und stellt einen zweiten Schutzwall gegen das Eindringen von Keimen und Fremdkörpern dar.

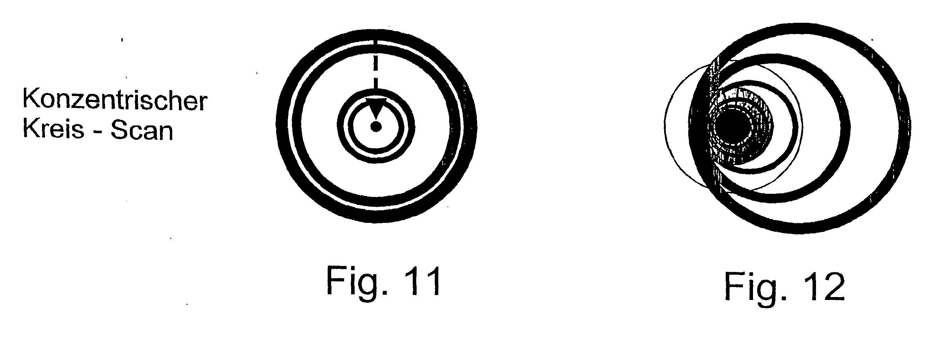

Aus der vorstehenden Beschreibung wird klar, dass das erfindungsgemäße Systemzur Aufnahme des Netzhautreflexbildes die Ausbildung einer Brille hat, mit deren Hilfe über eine Rückspiegelung an der Innenseite derselben das Netzhautreflexbild des Auges bei unterschiedlicher Helligkeit der Umgebung elektronisch aufgenommen, mit einem Computer modifiziert und über eine Beleuchtungseinrichtung und eine Rückreflexion über die gleiche Brille physiologisch verzögerungsfrei dem ursprünglichen Bild so überlagert wird, dass ein verbesserter Seheindruck entsteht. Anhand der Figuren 11 bis 14 wird eine modifizierte Ausführungsform des Systems beschrieben, das zur Realisierung der Anwendungen nach den Ansprüchen 1 bis 15 verwendet werden kann.

Die Verwendung von optoelektronischen Brillen zur Spiegelung von computergenerierten Bildern ins Auge mit den Namen "cyberspace" oder "virtual reality" nimmt heute rasant zu. Diese Technik ist sowohl für die Anwendung in der Unterhaltungsindustrie als auch in den verschiedenen Gebieten der Industrie, Verkehr und Medizin von breitem Nutzen und wird mit der Verfügbarkeit von immer schnelleren Bildverarbeitungscomputern an Verbreitung und Bedeutung ständig zunehmen.

Am weitesten verbreitet ist die Anwendung mit geschlossenen, nicht transparenten Brillen, bei der Bilder von miniaturisierten Kathodenstrahlröhren oder Flüssigkristall-Matrizen über Spiegel- oder Glasfasersysteme dem Auge dargeboten werden. Die besondere Attraktivität dieser Technik ist es, mit bewegter dreidimensionaler Bilddarstellung, den Bildablauf oder die Handlung mit verschiedenen Bewegungen des Brillenträgers zu koppeln. So wird eine Änderung der Blickrichtung durch Kopfbewegung oder Änderung der Perspektive bei fortschreitender Bewegung nachgebildet. Es können die Bewegungen der Arme und Finger des Brillenträgers mit Hilfe von Sensoren in das Bild eingebracht werden, um ihm die Möglichkeit des direkten Eingreifen in die Handlung zu ermöglichen.

In neueren Systemen mit dem Namen "augmented reality" kann der Brillenträger mit Hilfe teiltransparenter Brillen sowohl die Umgebung als auch ein über die Brille eingespiegeltes Bild von Kameras von der gleiche Szene oder von anderen Bildinhalten über einen miniaturisierten Monitor am Helm betrachten. Eine wohlbekannte Variante dieses Verfahrens ist bei der Führung von Kampfflugzeugen mit dem Namen Helmet-mounted-display (HMD) bereits eingeführt.

Bei dieser Techniken sind jedoch mehrere Probleme bekannt, die auf die Wirkungsweise des Gesichtsinnes zurückzuführen sind und auf verbesserte technische Lösungen warten. Bei einer geschlossenen Brille und einem starr gekoppelten Monitor und Monitorbild bewegt sich bei einer Kopfbewegung des Brillenträgers die Szene in gleicher Richtung mit, was seinen Sehgewohnheiten in unnatürlicher Weise widerspricht. Er ist durch die Abbildung des Auges gewohnt, dass die Szene gerade in entgegengesetzter Richtung verläuft. Dieses Problem konnte durch die umständliche Messung der Kopfbewegung und des Augapfels mit externen Drehwinkelsensoren, einer entsprechenden Bildverarbeitung und Nachführung des generierten Bildes bis jetzt nur unvollkommen gelöst werden.

Durch Anpassungsbewegungen des Augenapfels, die von sogenannten vestibulären okularen Reflexen (VOR) des Ohren-Bogengang-Systems ausgehen und dem Festhalten des Fixationspunktes bei Kopfbewegungen dient, ist das Auge selbst in der Lage, das Netzhautbild grob zu stabilisieren. Die Feineinstellung geschieht mit dem Bild als Referenz. Dieses Bild- Tracking wird zusätzlich vom Auge verwendet, um die VORs einer dynamischen Augenausrichtung zu adaptieren.

Dies bedeutet, dass eine Überlagerung von Fremdbildern erst bei ihrer Ankopplung an das reale Netzhautbild einen wirklichkeitsgetreuen Bildeindruck geben kann.

Bei geschlossenen Brillen wird versucht das Bild der Blutgefässe (Augenhintergrund) als Referenz zu verwenden (Retina-Tracking). Das liefert allerdings nur eine ungenügende Auflösung und ist ausschliesslich für monokulare Betrachtungen geeignet (siehe z. B. E.Peli, "Visual issues in the use of a head-mounted monocular display", Optical Engineering, vol. 29, No. 8, p. 883 (1990). Eine gleichzeitige Stabilisierung in beiden Augen von Bildern mit diesen ist wegen der unterschiedlichen Ausrichtung der Augen praktisch unmöglich. Neben der Verschlechterung der Bildqualität, führt der Konflikt zwischen den vestibulären und visuellen Informationen häufig zu Bewegungsstörungen bis hin zur Seekrankheit. Diese Probleme der bestehenden Technik werden z. B. in dem Übersichtsartikel von E. Peli, "Real Vision & Virtual Reality" in Optics & Photonics News, July 1995, S. 28-34 beschrieben.

Mit dem modifizierten System nach den Figuren 11 bis 14 werden die Probleme der Bildstabilisierung bei Überlagerung von Fremdbildern mit dem realen Bild gelöst.

Zwar sind mit der Technik des Systems gemäß den Figuren 1 bis 9 die vorgenannten Probleme grundsätzlich gelöst, jedoch sind konkrete Realisierungen und Anwendungen nicht angegeben. Der Grundgedanke der Variante nach den Figuren 11 bis 14 besteht darin, dieses Verfahren zur Verbesserung des Wahrnehmungsvermögens des Auges einzusetzen. Die physikalischtechnischen Probleme, die dazu gelöst werden müssen, ergeben si aus den physiologischen Eigenschaften des Auges und den ständig variierenden Beleuchtungsverhältnissen in der Umgebung. Das Auge ist wegen der variablen Lichtverhältnisse und der unterschiedlichen optischen Aufgaben in seinen Grundfunktionen ein sehr dynamisches Sinnesorgan. Es adaptiert sich an die Variation der Intensität der Hintergrundbeleuchtung über 12 Dekaden. Es schaltet vom Farbsehen beim Tageslicht auf reines schwarz/weiss Sehen in der Nacht um. Licht in dem Wellenlängenbereich 400-1500 nm wird von dem Auge transmittiert und auf die Netzhaut abgebildet. Dabei wird nur Licht im Bereich von 400 nm bis 750 nm wahrgenommen, d. h. das infrarote Licht im Bereich von 750-1500 nm, das sowohl bei Aussen- als auch Innenbeleuchtung sehr hell ist, bleibt für die visuelle Wahrnehmung ungenutzt.

Das Auge erfasst horizontal und vertikal einen Winkelbereich von etwa 100 DEG . Die Bildauflösung nimmt jedoch mit dem Winkelabstand von der Sehachse sehr schnell ab. Das aufmerksame momentane Sehen ist auf einen zentralen Winkelbereich von nur +/- 5 DEG begrenzt und das "scharfe" Sehen, z. B. beim Lesen oder Autofahren, ist auf den sehr geringen zentralen Winkelbereich von +/- 0,5 DEG begrenzt. Hinzu kommen ständig verschiedenartige Bewegungen der Augen. Dies führt zu folgenden Konsequenzen, die unter bestimmten Umständen das Wahrnehmungsvermögen des Auges beeinträchtigen und die im Rahmen der neuen Erfindung verbessert werden sollen:
- Adaption - Akkommodation - Schärfeleistung - Sehfehler - Altersbedingte Minderleistung und - Bewegungsdynamik.

Die der Variante nach den Figuren 11 bis 14 zugrundeliegende Aufgabe besteht darin, eine Anordnung bereitzustellen, die ähnlich wie das Auge in ihren Grundfunktionen sehr variabel gestaltet und an die Erfordernisse des Sehvorgangs angepasst ist, aber gleichzeitig die besondere Physiologie und Dynamik des Auges und die variierenden Beleuchtungsverhältnisse der Umgebung und den unsichtbaren IR-Bereich berücksichtigt und ausnutzt.

Ein grundsätzliches Problem der seriellen gegenüber der parallelen Bildabtastung ist die kurze Verweilzeit des Scanners in jedem Bildpixel.

Eine gleichmässige Abtastung z. B. von 0,5 Mio Bildpunkte in einer Abtastzeit von 40 ms bedeutet eine Integrationszeit von nur 0,08 mu s d. h. 80 ns in jedem Bildpunkt. Im Vergleich hierzu beträgt die parallele Zeitintegration aller Bildpunkte des Auges selbst 10-20 ms.

Wie aus der Anwendung von Lasern zur Aufnahme der Netzhautstruktur des Auges in den sogenannten Laser Scanning Ophthalmoskopen bekannt ist, ist eine Laserleistung von etwa 40 mu W notwendig, um beim Rasterscan ein Signal-Rausch-Verhältnis von 17 aus einem Bildpixel zu erzielen (siehe z. B. A.Plesch, U. Klingbeil, and J. Bille, "Digital laser scanning fundus camera", Applied Optics, Vol. 26, No 8. p. 1480-1486 (1987)). Umgerechnet auf die grössere Fläche würde dies einer Bestrahlungsstärke in einem Abbild einer ausgedehnten Quelle auf der Netzhaut von 40 W/cm<2> entsprechen, was der Bestrahlungsstärke von hellen Scheinwerfern oder Sonne auf der Netzhaut entspricht, d. h. mit dem Rasterscan können erst relativ helle Quellen mit einem guten Signal-Rauschverhältnis auf der Netzhaut aufgezeichnet werden. Um die Abbildung von schwächeren Quellen auf der Netzhaut zu detektieren, muss die Empfindlichkeit wesentlich gesteigert werden.

Die serielle Bildabtastung hat jedoch für die Aufnahme des Netzhautreflexes den entscheidenden Vorteil der besseren Unterdrückung von Streulicht, der einfacheren Aufnahmeoptik und der Möglichkeit der exakten Umkehrung des Strahlenganges bei der Bildrückprojektion mit einem Laser und soll aus diesen Gründen auch in dieser Erfindungsmeldung beibehalten werden. Eine Verlängerung der Verweilzeit kann aber durch Änderung des Scanmusters erreicht werden.

Wegen der ungleichmässigen Verteilung der Photorezeptoren, mit der höchsten Dichte der Zapfen für das scharfe Sehen im Zentrum der Netzhaut und dem entgegengesetzten Verlauf der Stäbchen für das unscharfe aber lichtempfindliche Nachtsehen, ist der Rasterscan keineswegs das optimale Scanmuster. Ein an den Sehvorgang angepasstes Scanmuster sollte für das Tagessehen in Richtung zum Zentrum zunehmend langsamer und dichter werden, für die Anpassung an das Nachtsehen gerade umgekehrt.

Ausser der Verweilzeit kann das aufgenommene Signal durch Änderung der Fleckgrösse der Abtastung und damit auch der Bildauflösung beeinflusst werden.

Die Anzahl der Signalphotonen Ns die von einem abtastenden Aufnahmegerät von der Netzhaut pro Bildpixel aufgenommen werden, kann nach der folgenden Formel berechnet werden: Ns = (B T DELTA lambda tau ) (AoR)(S/2)(Ap/D<2>) (1/ epsilon ) wobei B = die spektrale Bestrahlungsstärke auf der Netzhaut T = die optische Transmission von Netzhaut bis zum Photodetektor tau = die Integrationszeit in einem Bildpixel auf der Netzhaut Ao = die Fläche des Bildpixels R = Reflexionsvermögen des Bildpixels DELTA lambda = Spektrale Breite des Empfangssignals Ap = Pupillenfläche D = Abstand der Pupille zur Netzhaut S/2 = der Winkelverteilungsfaktor der optischen Rückstreuung der Netzhaut epsilon = Energie eines Photons bei der Aufnahmewellenlänge bezeichnen.

Wie diese Formel zeigt, können stärkere Signale, d. h. eine grössere Anzahl von Signalphotonen durch folgende Massnahmen am Aufnahmegerät gewonnen werden:
- Verlängerung der Verweildauer tau des Scans in den einzelnen Bildpunkten, - Vergrösserung des Abtastflecks Ao auf der Netzhaut - Vergrösserung der spektralen Bandbreite DELTA lambda .

Die Variante nach den Figuren 11 bis 14 schlägt das Abtasten der Netzhaut in einer Abfolge von konzentrischen Kreisen vor (Kreismittelpunkt ist gleich Fovea centralis), deren Radius sich sukzessive vergrössert bzw. verkleinert. Diese Art des Scannens wird als Kreisscan bezeichnet. Wegen der Rotationssymmetrie der Augenlinse und der Pupille um die Sehachse und der rotationssymmetrischen Verteilung der Photorezeptoren in der Netzhaut ist der Kreisscan optimal.

Die Erfindung schlägt weiterhin vor, dass ein identischer Kreisscan für die Aufnahme des Netzhautreflexes von der Umgebung und die Bildprojektion mit dem Laser verwendet werden. Da beim Kreisscan von aussen bis zum Zentrum, nach dem Erreichen des Zentrums, die Scanachse den gleichen Weg rückwärts verläuft, kann wahlweise die Aufnahme beim Scan bis zum Zentrum und Projektion von Zentrum bis aussen, oder die Aufnahme über den gesamten Abtastvorgang und Projektion erst in einem zweiten verwendet werden.

Bei einer konstanten Auslenkung von Scanspiegeln in zwei Richtungen (Lissajou-Figur) erfolgt beim Kreisscan zwangsläufig eine Verlangsamung der Verweildauer in Richtung zum Zentrum. Die Erfindung sieht jedoch vor, dass für das Tagessehen die Scandauer benachbarter Kreise, je nach den Belichtungsverhältnissen, noch zusätzlich verlangsamt und für das Nachtsehen sogar beschleunigt werden kann.

Aufgrund der ungleichmässigen Verteilung der Zapfen über die Netzhaut mit einer um mehr als zwei Dekaden höheren Dichte im Zentrum kann die Abtastrate (Verweildauer pro Bildpunkt) in diesem Bereich um diesen Faktor, 100 erhöht werden.

Für das Nachtsehen mit der höheren Verteilung der Stäbchen mit zunehmenden Radius ist es sinnvoll, dass die Verweildauer entgegengesetzt nach aussen in ähnlichem Masse abnimmt.

Wie dem Fachmann bekannt, lässt sich ein Kreisscan in analoger Ansteuerung mit periodisch schwingenden orthogonalen Scanspiegeln, oder in digitaler Ansteuerung durch Näherung an die Kreisspur mit einer hohen Anzahl von geraden Strecken. Als dritte Alternative bietet sich die Verwendung von programmierbaren Algorithmen analoger Ansteuersignale, die digital aufgerufen werden können und am besten für diese variablen Verhältnisse am geeignet sind.

Damit das Empfangssignal auch durch Vergrösserung des abgetasteten Bildflecks, proportional zu seiner Fläche, zusätzlich erhöht werden kann, sieht die Erfindung ferner vor, dass die momentane Bildpixelgrösse auf der Netzhaut zusätzlich zur Scangeschwindigkeit variabel eingestellt werden kann.

Mit der Änderung der Bildfleckgrösse wird auch die Bildauflösung entsprechend der Situation angepasst. Ausser der Veränderung der Abtastfläche kann die Auflösung durch variablen Radiensprung der Scanradien eingestellt werden.

Mit einer Vergrösserung des abtastenden Bildpixels von z. B. 10 mu m auf 100 mu m wird z. B. die Bildauflösung von etwa 2 bis 20 Bogenminuten (Auflösungsbereich des Lesens und Betrachtens) um einen Faktor 10 reduziert, gleichzeitig wird das empfangene Signal um einen Faktor 100 erhöht.

Wie dem Fachmann bekannt, ist bei der konfokalen Abtasten die Bildauflösung durch den Blendendurchmesser im Zwischenfokus vor dem Photodetektor bestimmt und kann durch seine Veränderung eingestellt werden. Die Erfindung sieht vor, dass hierfür Flüssigkristallblenden oder elektrooptische Blenden verwendet werden, damit diese Einstellung möglichst schnell, d. h. innerhalb eines Abtastzyklus, durchgeführt werden kann.

Da der zeitliche Ablauf des Scannens und die Grösse des Bildpixels bei Aufnahme und Projektion möglichst identisch sein sollten, schlägt die Erfindung vor, dass die die Änderung des Scanverlaufs und Blendenregelung im Projektionskanal die gleiche ist wie im Aufnahmekanal. Die Variation der optischen Integrationszeit und Bildpixelfläche kann dann im Projektionskanal durch entsprechende Variation der Sendeleistung des Lasers kompensiert werden.

Die Höhe des Empfangssignals ist weiterhin von der spektralen Bandbreite des Empfängers abhängig und kann durch ihre Verbreiterung erhöht werden. Die Erfindung sieht vor, dass im Bereich des hellen Tagessehen (photopisches Sehen) eine der Augenfarbempfindlichkeit entsprechende Aufspaltung des Strahlenganges in die Farbkanäle Rot-Grün-Blau mit je einer spektralen Breite von etwa 100 nm vorgenommen werden kann. Dies ermöglicht eine farbechte Bildaufnahme und mit entsprechenden dreifarbigen Lasern eine farbliche Zurückprojektion ins Auge.

Bei einer schwacher Umgebungsbeleuchtung, bei der Farben nicht mehr vom Auge wahrgenommen werden (scotopisches Sehen) sieht die Erfindung die Zusammenlegung aller Kanäle zu einem einzigen (schwarz/weiss) Empfangskanal ohne Farbauflösung vor. Weiterhin sieht die Erfindung vor, dass dieser Empfangskanal nicht nur den sichtbaren Bereich von 400-700 nm, sondern zusätzlich den nahen infraroten Bereich von 700-1000 umfasst.

Dies bringt zur Erhöhung des Empfangssignals bei schwacher Hintergrundbeleuchtung die folgenden Vorteile:
- das Auge hat zwischen 400-1000 nm volle Transparenz und bildet ein vergleichbares Bild zwischen 700-1000 nm wie zwischen 400-700 nm ab. - der Reflexionsgrad der Netzhaut zwischen 700-1000 nm beträgt R = 10-20% gegenüber R = 3-5% zwischen 400-700 nm - es sind Photoempfänger mit hohem Quantenwirkungsgrad wie Photomultiplier und Silizium-Avalanchedioden über den gesamten Spektralbereich von 400-1000 nm verfügbar. - Glühlampen, die zur Innenbeleuchtung von Gebäuden, bzw. im Freien zur Strassenbeleuchtung und bei Fahrzeugen verwendet werden, strahlen zwischen 700-1000 nm 10 mal mehr Licht ab als zwischen 400-700 nm. - das Reflexionsvermögen der Vegetation der Natur ist um einen Faktor 5-10 zwischen 700-1000 nm höher als zwischen 400-700 nm.

Wie diese Beispiele zeigen, ist bei schwacher Beleuchtung (Nachtsehen) eine nochmalige Erhöhung des Empfangssignals um einen Faktor 100 durch Erweiterung des Spektralbereiches möglich.

Die Erweiterung des spektralen Bereiches kann entweder in jedem Gerät fest installiert sein oder durch Wechseln von spektralen Filtern variabel gestaltet werden. Wird eine Farbdarstellung nicht gefordert, ist es sinnvoll, grünes Laserlicht für die Rückprojektion ins Auge, wegen der höchsten Empfindlichkeit und Kontrastwahrnehmung des Auges bei dieser Farbe, zu verwenden.

Zusätzliche Methoden zur Signalverbesserung, die hier eingesetzt werden können, sind die Integration mehrerer aufeinanderfolgender Bilder und die Bildkorrelation, z. B. Bilder der beiden Augen.

Insgesamt kann durch die Variation der zwei Parameter, der Verweilzeit des Scans in den Bildpixels und der Grösse des Bildflecks, mit Hinzunahme des infraroten Bereiches und der Verwendung von Bildkorrelation, eine gesamte Dynamik der Empfangssignale über sieben Dekaden erfasst werden.

Bei einer gesamten optischen Transmission des Empfangskanals von T = 0,2 (siehe Formel oben) umfasst der Empfangbereich dieses dynamischen Aufnahmesystems Bestrahlungsstärken auf der Netzhaut zwischen 10<-><5> W/cm<2> und 100 W/cm<2>, was dem Bereich der typischen Innen- und Aussenhelligkeit umfasst.

Wegen der langsamen und schnellen Augenbewegungen ist es notwendig, das Scansystem so zu gestalten, dass es der Änderung der Sehachse durch die Brille ständig folgt, d. h. dass die Symmetrieachse der Bildabtastung, sowohl bei der Aufnahme, als auch bei der Projektion, mit der Sehachse identisch ist.

Die Variante des Systems nach den Figuren 11 bis 14 sieht zur Lösung dieser Aufgabe vor, dass vor und nach der Abtastung des Netzhautreflexes bzw. der Bildprojektion ins Auge, eine Zentrierung des Kreisscans auf der Augenpupille durchgeführt wird. Dabei wird der grösste Abtastwinkel des Kreisscans so gewählt, dass bei einer Ablage der Scansymmetrieachse von der Sehachse die Aussenfläche des Augapfels, Sclera mit Regenbogenhaut und Pupillenöffnung von dem Kreisscan erfasst wird. Da diese Teile des Auges, die vom Aussenlicht gut ausgeleuchtet sind, nicht scharf, sondern diffus in der Bildzwischenebene des Photodetektors abgebildet werden, liefert das Empfangssignal hier keine Bildinformation, sondern eine integrale Anzeige über das optische Rückstreuvermögen der Vorlage.

Wenn die Empfangssignale über zeitlich gleich lange Abschnitte, z. B. Quadranten, aus jedem Kreis miteinander verglichen werden, sind sie nur dann von gleicher Höhe, wenn die Achse des Kreisscans identisch ist mit der Augenachse (Sehachse). Signalunterschiede, wegen der unterschiedlichen Rückstreuung aus Sklera, Regenbogenhaut und Pupillenöffnung, sind dann ein Mass über die Achsenablage und ihre Richtung. Nach einer Normierung mit dem gesamten Empfangssignal über jeden Kreis können diese Ablagesignale zur Einstellung der Nullstellung eines nächsten Kreisscans (Bias) verwendet werden. Somit kann eine ursprüngliche Ablage der Achsen mit jedem Kreisscan vermindert werden bis sie beim Eintauchen des Kreisscans durch die Pupillenöffnung verschwindend gering wird (Pupillentracking). Fig. 11 zeigt schematisch den kozentrischen Scanvorgang bei justierten System, Fig. 12 stellt den Suchmode der Zentrierung des Scans durch die Augenpupille dar.

Die Erfindung sieht alternativ zur Verwendung des Umgebungslichtes vor, dass auch mit der aktiven Beleuchtung der Laserprojektion ins Auge ein Pupillentracking im Aussenbezirken des Kreisscans, mit gleichzeitiger Signalauswertung im Aufnahmekanal wie oben beschrieben, durchgeführt wird.

Die Erfindung sieht ausserdem vor, dass auch während der Laserbildprojektion das sowohl von der Umgebung als auch vom Laser zurückgestreute Licht aufgenommen und ausgewertet wird. Diese gleichzeitige Aufnahme des Netzhautreflexes von der Umgebung und der nachbearbeitenden Laserbildprojektion eröffnet die Möglichkeit, den Grad der Überlappung und die zeitliche Synchronisation beider Bilder ständig zu überprüfen, eventuelle Unterschiede als Bildinterferenzen (Moiré-Muster) zu erkennen, um diese dann durch Korrektursignale nachträglich zu kompensieren.

Die Aufnahme und Projektionstechnik im Sinne der Erfindung kann entweder an einem Auge eines Betrachters oder an seinen beiden Augen gleichzeitig, unabhängig voneinander durchgeführt werden. Wegen des steroskopischen Sehens beider Augen wird in dem letzteren Fall eine dreidimensionale Bildaufnahme und Bildwiedergabe realisiert.

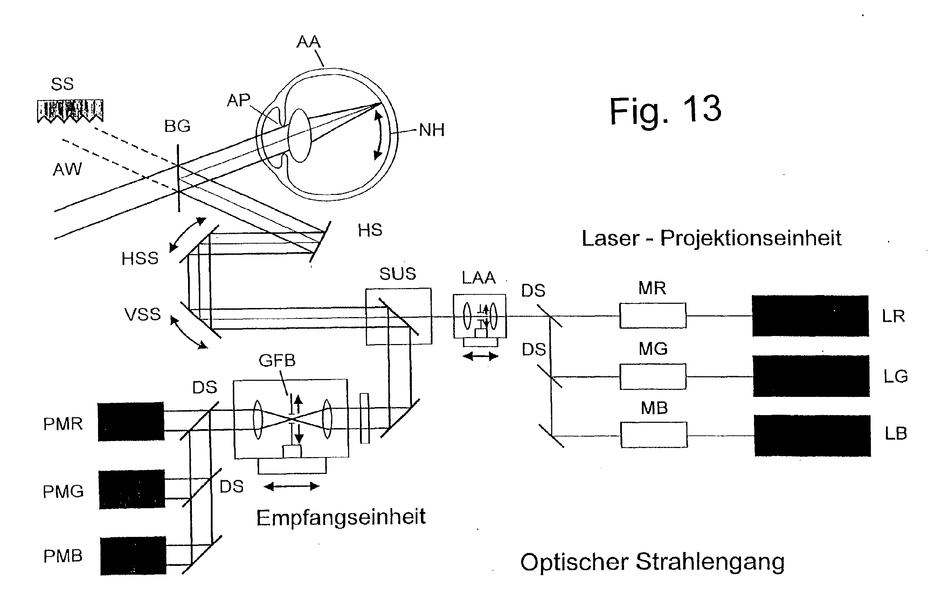

Es ist nicht ohne weiteres verständlich, dass die Aufnahme eines fehler- und verzeichungsfreien Reflexbildes der Umgebung von der Netzhaut über eine Brille, die weder in ihren optischen Eigenschaften individuell an jeden Betrachter angepasst, noch vollständig stabil sitzend auf dem Kopf des Betrachters sein kann. Die Lösung hierzu im Sinne der Erfindung besteht erstens in der relativ geringen optischen Anforderungen an den seriellen konfokalen Punktscan gegenüber z. B. einer flächenhaften Abbildung aus dem Auge, zweitens in der vollständigen dynamischen Anpassung des optischen Strahlenganges des Scanners über die Brille in das Auge, die jedesmal die Eigenbewegungen des Auges und der Brille selbst berücksichtigt, drittens in der exakten Rückkehrung des Strahlenganges zwischen Aufnahme und Projektion und der kurzen Zeitdauer zwischen diesen Vorgängen. Zur Einstellung des Scan durch das Auge, auch bei den verschiedenen Augenbewegungen dienen zwei scannende Elemente und ein Korrekturspiegel der auch justierbar sein kann. Die Fig. 13 zeigt schematisch in Übersicht das ganze System. Die Netzhaut des Auges NH wird mit dem fokussierten Strahl abgetastet. Hier stellt AA den Augapfel und AP die Augenpupille dar. Die teildurchlässige Brille ist hier mit BG bezeichnet.

Die von der Umgebung durchgehenden Strahlen werden auf der Netzhaut fokussiert, gleichzeitig wird die Netzhaut punktuell abgetastet, wobei der Abtaststrahl in Transmission durch die Brille immer gegen eine Strahlungssenke schaut. Mit den zweiachsigen Abtastelementen HSS und VSS wird der Kreisscan durchgeführt. Mit dem Hilfsspiegel HS, der aktiv einstellbar sein kann, wird die Einfallsrichtung und Position des Strahles auf der Innenfläche der Brille BG eingestellt. Mit dem Strahlumschalter SUS kann entweder mit einer zentralen Bohrung der beleuchtende Laserstrahl durchgelassen werden und der Empfangsstrahl, der meist wesentlich grösseren Durchmesser hat in die Empfangseinheit reflektiert werden in getrennte Richtungen geleitet werden, oder es kann ein aktiv umschaltendes Spiegelelement verwendet werden das zwischen Empfang und Senden umschaltet.

Die Empfangseinheit kann z. B. aus drei getrennten Empfangskanälen für die Grundfarben Rot, Grün und Blau oder andere Wellenlängenbereiche z. B. im nahen Infrarotbereich bestehen. Der Strahlengang aller Spektralkanäle wird mit Hilfe dichroitischer Spiegel DS auf eine Achse gebracht. Zur Einstellung der Fleckgrösse des Abtaststrahles auf der Netzhaut und zur eventuellen Feinkorrektur der optischen Achse dient eine aktiv justierbare Gesichtsfeldblende GFB.

Die Sendeeinheit kann z. B. aus drei Lasern mit den Grundfarben Rot LR, Grün LG und Blau LB geschaffen sein. Vor der Strahlvereinigung auf einer Achse mit dichroitischen Spiegeln DS werden die Einzelnen Strahlen entweder extern mit Bildmodulatoren MR, MG und MB moduliert oder einfacher direkt über den Anregungsstrom der Laseremission. Die Grösse und Lage des Laserabtastflecks auf der Netzhaut wird mit einer aktiv steuerbaren Blende LAA die im Zwischenfokus zweier Linsen im Strahlengang eingestellt wird. Als Empfänger für die Abtastung der Netzhautreflexbildes sind z. B. Photomultiplier geeignet, die wechselweise bei sehr schwachen optischen Signalen in einen Photon-counting Betrieb und bei starken Signalen in einen Strommessbetrieb automatisch Umschalten. Auch ist die Verwendung von Avalanche Photodioden als Empfänger möglich.

Als Lichtquellen zur Rückprojektion der Bilder ins Auge sind Halbleiterlaser bzw. miniaturisierte Festkörperlaser vorgesehen mit einer niedriger Dauerstrichleistung (< 300 mu W), die keine Gefährdung des Auges verursachen können. Mit der Verwendung von Halbleiterlasern könnte die Bildmodulation direkt über ihre Stromversorgung durchgeführt werden. Damit aller Farben erzeugt werden empfiehlt sich die Verwendung von drei Lasern mit den Grundfarben rot, grün und blau. Wie das bekannte Farbdreieck des menschlichen Gesichtsinnes zeigt können alle anderen Farben sowie die Unfarben grau und weiss durch Farbsummation von monochromatischen Laserlinien dieser Farben gebildet werden. Die Erfindung beinhaltet auch die Möglichkeit der Verwendung von einzelnen Farben als monochromatische Lösung vor.

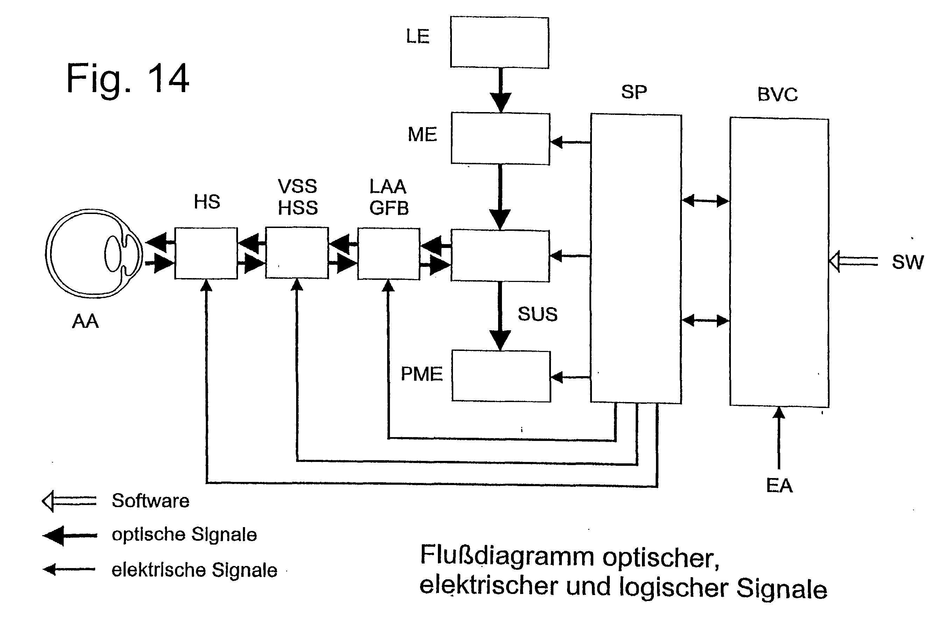

Die Erfindung sieht wie in Fig. 14 dargestellt einen Signal-Prozessor SP vor, der das direkte Bild von der Netzhaut elektronisch bearbeitet und alle Funktionen der Vorrichtung sowie die von Scannern VSS/HSS, des Hilfsspiegels HS und Laserfleckeinstellung LAA und Grösse der Gesichtsfeldblende GFB synchron koordiniert. Der Bildverarbeitungscomputer BVC übernimmt dann die vom Auge wahrgenommenen Bild oder Bilder anderer technischer Sensoren, die über einen externen Anschluss EA dem Computer zugeführt werden und bearbeitet sie nach einer vorgegebenen Software SW, bevor sie mit Hilfe des Signalprozessors auf die Laserstrahlen als Bildsignal aufmoduliert werden. In Fig. 14 sind der Fluss der optischen und elektrischen sowie der Softwaresignale getrennt dargestellt. Die komplette Lasereinheit wird mit DE bezeichnet, ME als Modulationseinheit und PME die komplette Empfangseinheit und SUS als der Strahlumschalter zwischen der Sende- und Empfangseinheit.

Die Laser-Projektion ermöglicht ausser der Verarbeitung des aktuellen vom Computer verarbeitetes Bild in das Auge zu projizieren und mit dem Originalbild zu verschmelzen, auch Fremdbilder, die dem Computer von extern zugeleitet werden, dem Aussenbild im Auge synchron zu überlagern. Wenn die Zeitspanne zwischen Bildaufnahme- und Projektion im Vergleich zu den schnellen Augebewegungen entsprechend kurz ist, wird das Auge, wie bei der Betrachtung eines Fernsehschirmes, keine Bildunterbrechung mehr wahrnehmen.

Die getrennte aber gleichzeitige Bildabtastung an beiden Augen erfasst auch die perspektivischen Unterschiede beider Bilder. Da diese bei der Laserzurückprojektion in beiden Augen erhalten bleiben, ist eine Wiederherstellung des räumlichen Sehens gewährleistet.

Die in der Erfindung verwendeten Bauelemente sind heute weitgehend miniaturisiert und kostengünstig erhältlich. Zum Scannen der Kreisfiguren können miniaturisierte Kippspiegel verwendet werden. Als zweite Möglichkeit zur Herstellung der Kreisfiguren bietet sich an die Verwendung von Keilplatten-Scannern, die für einen Strahlengang in Transmission ausgelegt sind. Der durchgehende Strahl wird durch jede der Platten um einen festen Winkel gebrochen, der gesamte Ablenkwinkel kann dann durch eine feste Drehung der Keilplatten gegeneinander kontinuierlich bis Null eingestellt werden. Bei einer gemeinsamen Drehung der Keilplatten mit einer festen Drehfrequenz beschreibt der abgelenkte Strahl dann eine Kreisspur. Als dritte Möglichkeit bietet sich die Verwendung von akusto-optischen Ablenkeinheit, die den Vorteil der geringen Trägheit und der schnellen Ablenkung bieten. Der variabel einstellbare Hilfsspiegel HS wird vorzugsweise als einer mit Mikroaktorik in zwei Achsen einstellbarer Spiegel sein.

Für die Einstellung des Laserfleckgrösse und des Empfangsgesichtsfeldes bieten sich vorzugsweise mikromechanische Aktoren wie z. B. in den weitverbreiteten Laser-Printer und CD-Plattenspielern auch verwendet werden.

Die Strahlumlenkeinheit und Scanner können ein einem einfachen Brillengestell untergebracht werden. Mit Hilfe von Glasfaserleitung können Laserprojektionseinheit in einem kleinen Gehäuse z. B. in der Grösse eines Taschenbuches mit Betterieversorgung untergebracht werden. Der Datenaustausch mit einem externen fest installierten Bildverarbeitungsrechner kann entweder über Radiowellen oder Infrarotstrahle erfolgen. Alle Elemente der Vorrichtung der Erfindung könnten nach dem heutigen Stand der Technik somit von einem Menschen mühelos getragen werden und der drahtlose Bilddatenaustausch mit dem externen Rechner würde seine unbeschränkte Bewegungsfreiheit ermöglichen.

Die Erfindung läßt sich wie folgt zusammenfassen:
1. System zur Aufnahme des Netzhautreflexbildes mittels eines Scansystems zum Abtasten eines Bildes auf der Netzhaut und zur Eispeisung von optischen Zusatzsignalen in das Auge, bei dem
   ein Strahlenteiler zur Transmission von Strahlen der Außenwelt in das Auge und zur Reflexion der von der Netzhaut des Auges zurückgestreuten Strahlen,
   eine Empfangseinheit zur Aufnahme der zurückgestreuten Strahlen und
   eine Projektionseinheit zur Projektion von Lichtstrahlen in das Auge verwendet werden, um auf der Netzhaut eine Abbildung zu erzeugen, die sich dem ursprünglich auf der Netzhaut abgebildeten Bild überlagert, wobei
   das Scansystem beim Abtasten die von der Netzhaut kommenden Strahlen umlenkt und zu einem opto-elektronischen Detektor zur seriellen Aufnahme des Netzhautreflexes weiterleitet,
   **gekennzeichnet durch** die Verwendung und/oder Ausbildung des Systems zur ophthalmalogischen Analyse, Therapie und/oder Diagnose.
2. System nach Punkt 1, **dadurch gekennzeichnet, daß** die Symmetrieachse des Scansystems der Sehachse nachgeführt wird.
3. System nach Punkt 2, **dadurch gekennzeichnet, daß** die beim Abtasten aufgrund der unterschiedlichen Rückstreuung aus Sklera, Regenbogenhaut und Pupillenöffnung auftretenden Signalunterschiede als Maß der Achsenablage und Achsenrichtung dienen.
4. System nach einem der Punkte 1-3, **gekennzeichnet durch** die Verwendung und/oder Ausbildung zur Analyse des Sehvermögens eines Patienten, indem mittels der Projektionseinheit auf der Netzhaut bzw. auf ausgewählten Bereichen der Netzhaut ein vorbestimmtes Muster bzw. eine vorbestimmte Musterverteilung generiert wird.
5. System nach Punkt 4, **gekennzeichnet durch** die Verwendung und/oder Ausbildung zur Analyse der Bewegungsmuster und/oder der Rauschfelder und/oder des räumlichen Sehvermögens eines Auges eines Patienten, indem für Prüfzwecke mittels der Projektionseinheit auf der Netzhaut Random-Dot-Muster generiert werden.
6. System nach einem der Punkte 1-3, **gekennzeichnet durch** die Verwendung und/oder Ausbildung zur Bestimmung von Anomalien der Augapfel-Motorik, indem in das System eine Einrichtung zur Bestimmung und Überwachung der Lage und/oder Orientierung des Augapfels integriert ist.
7. System nach Punkt 6, **gekennzeichnet durch** die Verwendung und/oder Ausbildung zur Bestimmung des Schielwinkels, indem eine Einrichtung zur Bestimmung und Überwachung des Augenmittelpunkts beider Augen integriert ist.
8. System nach Punkt 6, **gekennzeichnet durch** die Verwendung und/oder Ausbildung zur Aufdeckung von parasysympathischen/sympathischen Efferenzen, indem die Pupillomotorik mittels einer Detektoreinrichtung überwacht und ausgewertet wird.
9. System nach Punkt 6, **gekennzeichnet durch** die Ausbildung als Synoptophor oder Synoptometer ohne Apparatekonvergenz.
10. System nach Punkt 6, **gekennzeichnet durch** die Ausbildung als Einrichtung zur Bestimmung der Zyklodeviation.
11. System nach Punkt 6, **gekennzeichnet durch** die Ausbildung als Phasendifferenzhaploskop.
12. System nach Punkt 6, **gekennzeichnet durch** die Ausbildung als Einrichtung zur sichtachsenidentischen Detektion von Pnorien bei unterschiedlichen Blickrichtungen.
13. System nach einem der Punkte 1-3, **gekennzeichnet durch** die Verwendung und/oder Ausbildung zur Funktionsprüfung der Netzhaut, unter Heranziehung eines Muster-Elektro-Retinogramms (ERG) und einer Korrellationseinrichtung, mit der ein auf die Netzhaut gespieltes Bild in Korrellation mit dem tatsächlich ermittelten ERG bringbar ist.
14. System nach einem der Punkte 1-3, **gekennzeichnet durch** die Verwendung und/oder Ausbildung zur Messung der Kontrast-Empfindlichkeit des Sehvermögens eines Patienten vorzugsweise in Abhängigkeit von der Ortsfrequenz.
15. System nach einem der Punkte 1-3, **gekennzeichnet durch** die Verwendung und/oder Ausbildung zur Rauschfeldampimetrie.
16. System nach einem der Punkte 1-3, **gekennzeichnet durch** die Verwendung und/oder Ausbildung zur Bestimmung der Ausdehnung und der Lage zentraler Gesichtsfelddefekte (Skotome).
17. System nach einem der Punkte 1-3, **gekennzeichnet durch** die Verwendung und/oder Ausbildung als VEP (Visual Enabling for Precision Surgery)-Gerät.
18. System nach einem der Punkte 1-3, **gekennzeichnet durch** die Verwendung und/oder Ausbildung als SLO (Scanning Laser Ophthalmoloskop)-Gerät.
19. System nach einem der Punkte 1 bis 18 als Vorrichtung zur Aufnahme des auf der menschlichen Netzhaut des Auges abgebildeten Bildes der Aussenwelt mit Hilfe einer scannenden Aufnahmevorrichtung, **dadurch gekennzeichnet**, dass die Strahlen der Aussenwelt durch die Gläser einer Brille transmittiert werden, die auf ihren Innenseiten als gewölbte abbildende Strahlteilerspiegel gestaltet sind und dass zur Aufnahme des von jedem Punkt der Netzhaut zurückgestreuten und aus dem Auge austretenden parallelen Strahlenbündel und zu ihrer Abbildung und Umlenkung eine zweiachsige Scanvorrichtung vorgesehen ist, die das Lichtbündel zu einem opto-elektronischen Detektor zur seriellen Aufnahme des Netzhautreflexes weiterleitet.
20. System nach Punkt 19, **dadurch gekennzeichnet,** dass mit Hilfe eines Strahlteilers Laserstrahlen über den gleichen Lichtweg, die gleiche Scanvorrichtung sowie Abbildung und Umlenkung an der Innenseite der gewölbten Brillengläser in umgekehrter Richtung wie das Reflexbild auf die Netzhaut abgebildet werden.
21. System nach Punkt 19 oder 20, **dadurch gekennzeichnet,** dass mit Hilfe eines Strahlumschalters Laserstrahlen über den gleichen Lichtweg, die gleiche Scanvorrichtung sowie Abbildung und Umlenkung an der Innenseite der gewölbten Brillengläser in umgekehrter Richtung wie das Reflexbild auf die Netzhaut abgebildet werden.
22. System nach Punkt 19 bis 21, **dadurch gekennzeichnet,** dass die momentane optische Scanachse von Scanner aus gesehen in ihrer Verlängerung durch das Brillenglas immer in eine lichtabsorbierende Strahlungssenke verläuft.
23. System nach Punkt 19 bis 22, **dadurch gekennzeichnet,** dass die Abbildung und Umlenkung der von Scanner ausgehenden Strahlenbündel vor der Umlenkung der Brille ins Auge mit Hilfe eines konkaven Hilfsspiegels abgebildet und umgelenkt werden.
24. System nach Punkt 19 bis 23, **dadurch gekennzeichnet,** dass die Abbildung der von Scanner ausgehenden Strahlbündel vor der Umlenkung der Brille ins Auge sowohl mit Hilfe eines konkaven als auch konvexen Hilfsspiegels abgebildet und umgelenkt werden.
25. System nach Punkt 19 und 20, **dadurch gekennzeichnet,** dass die Bildabtastung und Laserprojektion im Auge gegeneinander zeitlich getrennt und alternierend mit einer festen Bildfrequenz durchgeführt werden.
26. System nach Punkt 19 bis 21, **dadurch gekennzeichnet,** dass während der Periode der Bildabtastung zeitweise auch eine Laserbildprojektion ins Auge durchgeführt wird.
27. System nach Punkt 19 bis 22, **dadurch gekennzeichnet,** dass die Abtastspur über die Netzhaut entsprechend den bekannten Video-Normen mit der Ablenkeinheit geführt wird.
28. System nach den Punkten 19 bis 27, **dadurch gekennzeichnet,** dass die Abtastspur rasterförmig über die Netzhaut mit einer entsprechenden Auslegung und Ansteuerung der Scanvorrichtung durchgeführt wird.
29. System nach Punkt 19 bis 27, **dadurch gekennzeichnet,** dass die Abtastspur spiralförmig über die Netzhaut mit einer entsprechende Auslegung und Ansteuerung der Scanvorrichtung geführt wird.
30. System nach Punkt 19 bis 29, **dadurch gekennzeichnet,** dass im optischen Empfangskanal mit Hilfe von mehreren Strahlteilern und Photodetektoren mehrere spektrale Bereiche des Empfangssignals unabhängig voneinander detektiert werden können.
31. System nach Punkt 19 bis 30, **dadurch gekennzeichnet,** dass im Beleuchtungskanal mit Hilfe von Strahlteilern mehrere Laserstrahlen auf die gleiche Strahlachse gebracht werden können und dadurch gemeinsam auf die Netzhaut abgebildet werden können.
32. System nach Punkt 19 bis 31, **dadurch gekennzeichnet,** dass der Strahlengang der Laserprojektionseinheit und Empfangseinheit mit einer starren Strahlführung ausgelegt ist.
33. System nach Punkt 19 bis 31, **dadurch gekennzeichnet,** dass der Strahlengang der Laserprojektionseinheit mit Lasern, Bildmodulatoren und Strahlteilern über eine Glasfaser an den Strahlumschalter geführt wird.
34. System nach Punkt 19 bis 32, **dadurch gekennzeichnet,** dass der Strahlengang der Empfangseinheit mit Photoempfängern und Strahlteilern über eine Glasfaser an die Strahlumschalteinheit geführt wird.
35. System nach Punkt 19 bis 34, **dadurch gekennzeichnet,** dass im Strahlengang der Laserprojektionseinheit eine Strahlfokussiereinrichtung integriert ist womit die Grösse des Bildflecks der Lase auf der Netzhaut variiert werden kann.
36. System nach Punkt 19 bis 35, **dadurch gekennzeichnet,** dass im Strahlengang der Empfangseinheit eine variable Gesichtsfeldblende integriert ist womit die Grösse des Abtastflecks auf der Netzhaut variiert werden kann.
37. System nach Punkt 19 bis 36, **dadurch gekennzeichnet,** dass das von den Photoempfängern aufgenommene Bild einem Bildverarbeitungscomputer zugeleitet wird der synchron mit der Bildabtastung die aufgenommenen Bilder bearbeitet.
38. System nach Punkt 19 bis 37, **dadurch gekennzeichnet,** dass das von einem Bildverarbeitungscomputer verarbeitete Bild der Netzhaut, mit Hilfe von elektro-optischen Modulatoren im Strahlengang der einzelnen Laserstrahlen ein Bild synchron aber zeitversetzt mit der Abtast auf der Netzhaut entstehen lässt.
39. System nach Punkt 19 bis 38, **dadurch gekennzeichnet,** dass Fremdbilder von anderen Sensoren als dem Auge in dem Bildverarbeitungscomputer mit dem dort abgespeicherten momentanen Netzhautb örtlich und zeitlich synchronisiert wird und mit den Laserstrahlen zeitversetzt ins Auge projiziert werden.
40. System nach Punkt 19 bis 39, **dadurch gekennzeichnet,** dass Informationen im Computer generiert in dem Bildverarbeitungscomputer mit dem dort abgespeicherten momentanen Netzhautbild örtlich und zeitlich synchronisiert wird und mit den Laserstrahlen ins Auge projiziert werden.
41. System nach Punkt 19 bis 40, **dadurch gekennzeichnet,** dass mit Hilfe eines optischen Schalters das Aussenlicht vor der Brille teilweise oder ganz zusperrt.
42. System nach Punkt 19 bis 40, **dadurch gekennzeichnet,** dass bei verminderter oder gesperrter Sicht nach aussen und bei Projektion von Laserbildern von fremden Sensoren oder Computern auf der Netzhaut auch während der Laserprojektion diese Bilder von den Detektoren aufgenommen und dem Bildverarbeitungscomputer zugeführt werden.
43. System nach Punkt 19 bis 42, **dadurch gekennzeichnet,** dass die von der Netzhaut aufgenommenen Bilder der Aussenwelt durch Umklappen des Strahlenganges um 180 DEG gegenüber dem Achsenverlauf ins Auge den Strahl in die Aussenwelt zur Beleuchtung der von dem Auge gesehenen Objekte mit dem vom Rechner abgeleiteten Laserbild verwenden.
44. System, nach einem der Punkte 19 bis 43 bei dem das Reflexbild in Inneren des Auges abgetastet wird und nach Modifikation auf dem gleichen Weg in das Auge zurückprojiziert wird, gekennzeichnet durch Verwendung eines Ellipsenscans.
45. System nach Punkt 44, **dadurch gekennzeichnet,** dass der Ellipsenscan durch Ermittlung der Aussenränder der Pupille zur Justierung und Zentrierung des Scannersystems ohne weitere externe Sensoren verwendet wird.
46. System nach Punkt 1, **dadurch gekennzeichnet,** dass das aufgenommene Bild mit dem zurückprojizierten Bild zeitlich und örtlich synchronisiert wird.
47. System nach Punkt 45, **dadurch gekennzeichnet,** dass die Scandauer dynamisch an die Anforderung von Auflösung, Erfasszeitbedarf und Belichtungszeit angepasst wird.
48. System nach Punkt 47, **dadurch gekennzeichnet,** dass die Grösse des Abtastfleckens dynamisch entsprechend der Anforderung der Umgebungsbedingungen angepasst wird.
49. System nach einem der Punkte 47 oder 48, **dadurch gekennzeichnet,** dass der Spurabstand der Scanspuren dynamisch entsprechend der Anforderung der Umgebungsbedingungen angepasst wird.
50. System nach Punkten 44 bis 49, **dadurch gekennzeichnet,** dass die Grösse des abgetasteten Bereichs entsprechend der Anforderungen des Anwendungsfalles angepasst wird.
51. System nach Punkt 44, **dadurch gekennzeichnet,** dass das aufgenommene Bild durch ein nachgeschaltetes Bildverarbeitungssystem aufgehellt wird und dann zurückprojiziert wird.
52. System nach Punkt 44 und 51, **dadurch gekennzeichnet,** dass das aufgenommene Bild durch eine Projektion des Bildes bei einer anderen Wellenlänge als es aufgenommen wurde und dadurch in einen anderen Lichtwellenlängenbereich transformiert wird.
53. System nach Punkt 44 und 52, **dadurch gekennzeichnet**, dass der Wellenbereich des aufgenommenen Bildes ausserhalb des Wahrnehmungsbereiches des Auges ausgewertet wird und in den sichtbaren Bereich transformiert wird.
54. System nach Punkt 44 und 51, **dadurch gekennzeichnet,** dass das aufgenommene Bild so stark aufgehellt wird, dass die ursprünglich vom Auge erkennbare Schwarz-Weiss (Stäbchensehen)- Information in eine Farbinformation transformiert wird (Zäpfchensehen).
55. System nach Punkt 44, **dadurch gekennzeichnet,** dass das aufgenommene Bild durch Berechnen und Modulieren der Projektion über einen geeigneten Algorithmus (Fouriertransformation) so geschärft wird, dass Sehfehler des Auges ausgeglichen werden.
56. System nach Punkt 44, **dadurch gekennzeichnet,** dass für die Justierung des Scannersystems ein externer Sensor zur Ermittlung der Pupillenposition verwendet wird.
57. System nach Punkt 44 bis 56, **dadurch gekennzeichnet,** dass das aufgenommene Bild hinsichtlich des Bildinhaltes ausgewertet wird um externe Reaktionen und Steuerfunktionen zu aktivieren.
58. System nach Punkt 57, **dadurch gekennzeichnet,** dass die Bildinhalte beider Augen verglichen werden.
59. System nach Punkt 57, **dadurch gekennzeichnet,** dass die Position der Pupillen beider Augen verglichen wird.
60. System nach Punkt 58, **dadurch gekennzeichnet,** dass der Bildinhalt der Forea Centralis beider Augen verglichen wird.
61. System nach Punkt 59 und 60, **dadurch gekennzeichnet,** dass die Position der Pupillen und Bildinhalte der Forea Centralis beider Augen zur Ermittlung der Sehachse verwendet werden für Triangolation (Entfernungsbestimmung).
62. System nach Punkt 44 bis 61, **dadurch gekennzeichnet,** dass die Bildinformation des Auges verwendet wird um die absolute Helligkeit der Umgebung zu ermitteln.
63. System nach Punkt 44-62, **dadurch gekennzeichnet,** dass die Bildinformation des Auges verwendet wird um die absolute Farbtemperatur des Lichtes zu ermitteln.
64. System nach Punkt 44 und 45, **dadurch gekennzeichnet,** dass das System zur Ermittlung der Pupillengrösse verwendet wird.
65. System nach Punkt 51, **dadurch gekennzeichnet,** dass das aufgenommene Bild durch ein nachgeschaltetes Bildverarbeitungssystems so aufgehellt wird, dass die physiologische Scheinempfindlichkeit in einen anderen weniger empfindlichen Bereich verschoben wird.
66. System nach einem der Punkte 44 bis 65, **dadurch gekennzeichnet,** dass der Ellipsenscan von aussen nach innen läuft.
67. System nach einem der Punkte 44 bis 65, **dadurch gekennzeichnet,** dass der Ellipsenscan von innen nach aussen läuft.
68. System nach einem der Punkte 44 bis 65, **dadurch gekennzeichnet,** dass der Ellipsenscan durch Zusammenfallen der beiden Brennpunkte zu einem Kreisscan verändert wird.

## Patentansprüche

1. System zur Aufnahme des Netzhautreflexbildes mittels eines Scansystems zum Abtasten eines Bildes auf der Netzhaut und zur Einspeisung von optischen Zusatzsignalen in das Auge, bei dem
ein Strahlenteiler zur Transmission von Strahlen der Außenwelt in das Auge und zur Reflexion der von der Netzhaut des Auges zurückgestreuten Strahlen,
eine Empfangseinheit zur Aufnahme der zurückgestreuten Strahlen und
eine Projektionseinheit zur Projektion von Lichtstrahlen in das Auge verwendet werden, um auf der Netzhaut eine Abbildung zu erzeugen, die sich dem ursprünglich auf der Netzhaut abgebildeten Bild überlagert, wobei
das Scansystem beim Abtasten die von der Netzhaut kommenden Strahlen umlenkt und zu einem opto-elektronischen Detektor zur seriellen Aufnahme des Netzhautreflexes weiterleitet,
**gekennzeichnet durch** die Verwendung und/oder Ausbildung des Systems zur ophthalmalogischen Analyse, Therapie und/oder Diagnose.

2. System nach Anspruch 1, **dadurch gekennzeichnet, daß** die Symmetrieachse des Scansystems der Sehachse nachgeführt wird.

3. System nach Anspruch 2, **dadurch gekennzeichnet, daß** die beim Abtasten aufgrund der unterschiedlichen Rückstreuung aus Sklera, Regenbogenhaut und Pupillenöffnung auftretenden Signalunterschiede als Maß der Achsenablage und Achsenrichtung dienen.

4. System nach einem der Ansprüche 1-3, **gekennzeichnet durch** die Verwendung und/oder Ausbildung zur Analyse des Sehvermögens eines Patienten, indem mittels der Projektionseinheit auf der Netzhaut bzw. auf ausgewählten Bereichen der Netzhaut ein vorbestimmtes Muster bzw. eine vorbestimmte Musterverteilung generiert wird.

5. System nach Anspruch 4, **gekennzeichnet durch** die Verwendung und/oder Ausbildung zur Analyse der Bewegungsmuster und/oder der Rauschfelder und/oder des räumlichen Sehvermögens eines Auges eines Patienten, indem für Prüfzwecke mittels der Projektionseinheit auf der Netzhaut Random-Dot-Muster generiert werden.

6. System nach einem der Ansprüche 1-3, **gekennzeichnet durch** die Verwendung und/oder Ausbildung zur Bestimmung von Anomalien der Augapfel-Motorik, indem in das System eine Einrichtung zur Bestimmung und Überwachung der Lage und/oder Orientierung des Augapfels integriert ist.

7. System nach Anspruch 6, **gekennzeichnet durch** die Verwendung und/oder Ausbildung zur Bestimmung des Schielwinkels, indem eine Einrichtung zur Bestimmung und Überwachung des Augenmittelpunkts beider Augen integriert ist.

8. System nach Anspruch 6, **gekennzeichnet durch** die Verwendung und/oder Ausbildung zur Aufdeckung von parasysympathischen/sympathischen Efferenzen, indem die Pupillomotorik mittels einer Detektoreinrichtung überwacht und ausgewertet wird.

9. System nach Anspruch 6, **gekennzeichnet durch** die Ausbildung als Synoptophor oder Synoptometer ohne Apparatekonvergenz.

10. System nach Anspruch 6, **gekennzeichnet durch** die Ausbildung als Einrichtung zur Bestimmung der Zyklodeviation.

11. System nach Anspruch 6, **gekennzeichnet durch** die Ausbildung als Phasendifferenzhaploskop.

12. System nach Anspruch 6, **gekennzeichnet durch** die Ausbildung als Einrichtung zur sichtachsenidentischen Detektion von Pnorien bei unterschiedlichen Blickrichtungen.

13. System nach einem der Ansprüche 1-3, **gekennzeichnet durch** die Verwendung und/oder Ausbildung zur Funktionsprüfung der Netzhaut, unter Heranziehung eines Muster-Elektro-Retinogramms (ERG) und einer Korrellationseinrichtung, mit der ein auf die Netzhaut gespieltes Bild in Korrellation mit dem tatsächlich ermittelten ERG bringbar ist.

14. System nach einem der Ansprüche 1-3, **gekennzeichnet durch** die Verwendung und/oder Ausbildung zur Messung der Kontrast-Empfindlichkeit des Sehvermögens eines Patienten vorzugsweise in Abhängigkeit von der Ortsfrequenz.

15. System nach einem der Ansprüche 1-3, **gekennzeichnet durch** die Verwendung und/oder Ausbildung zur Rauschfeldampimetrie.

16. System nach einem der Ansprüche 1-3, **gekennzeichnet durch** die Verwendung und/oder Ausbildung zur Bestimmung der Ausdehnung und der Lage zentraler Gesichtsfelddefekte (Skotome).

17. System nach einem der Ansprüche 1-3, **gekennzeichnet durch** die Verwendung und/oder Ausbildung als VEP (Visual Enabling for Precision Surgery)-Gerät.

18. System nach einem der Ansprüche 1-3, **gekennzeichnet durch** die Verwendung und/oder Ausbildung als SLO (Scanning Laser Ophthalmoloskop)-Gerät.
